# EUROPEAN PATENT APPLICATION

(11) **EP 4 321 179 A1**
(43) Date of publication of application: **14.02.2024**
(21) Application number: 22798507.4
(22) Date of filing: 30.01.2022
(51) Int. Cl.: A61K 47/64, A61K 47/62, A61K 47/68, A61K 47/34, A61P 35/00

(54) **METHOD FOR INITIATING POLYMERIZATION OF AMINO ACID N-CARBOXYANHYDRIDES IN AQUEOUS PHASE**

(30) Priority: 07.05.2021 CN 202110495254
(71) Applicant: Peking University, Beijing 100871 (CN)
(72) Inventor: LU, Hua, Beijing 100871 (CN); HU, Yali, Beijing 100871 (CN); ZHAO, Ruichi, Beijing 100871 (CN)
(74) Representative: Cabinet Becker et Associés
(86) International application number: PCT/CN2022/075144
(87) International publication number: WO 2022/233165

(57) **Abstract**

A method for initiating the polymerization of amino acid N-carboxyanhydrides in the water phase, that is, a method for polymerizing amino acid N-carboxyanhydrides, which comprises adding, in the presence of an initiator, one or more amino acid N-carboxyanhydrides in a mixed solution of water or water and an organic solvent for reaction to produce a polyamino acid. The initiator is used to initiate the polymerization of the amino acid N-carboxyanhydrides to generate a polyamino acid, and the organic solvent is selected from among the following group: acetonitrile, pyridine, N,N-dimethylformamide, tetrahydrofuran or dimethylsulfoxide. The described method can produce a polyamino acid in a short period of time at a high yield and by using a means in which polymerization can be controlled, and can be used to prepare a conjugate of functional molecules, such as proteins, polypeptides, drugs, and fluorescent dyes, and a polyamino acid.

## Description

This application claims priority to Chinese Patent Application No. 202110495254.5, which was filed on May 07, 2021 and entitled "METHOD FOR INITIATING POLYMERIZATION OF AMINO ACID N-CARBOXYANHYDRIDES IN AQUEOUS PHASE".

### FIELD OF THE INVENTION

The present disclosure relates to a method of polymerizing amino acids, and specifically to a method of initiating the polymerization of amino acid N-carboxyanhydrides in an aqueous phase.

### BACKGROUND OF THE INVENTION

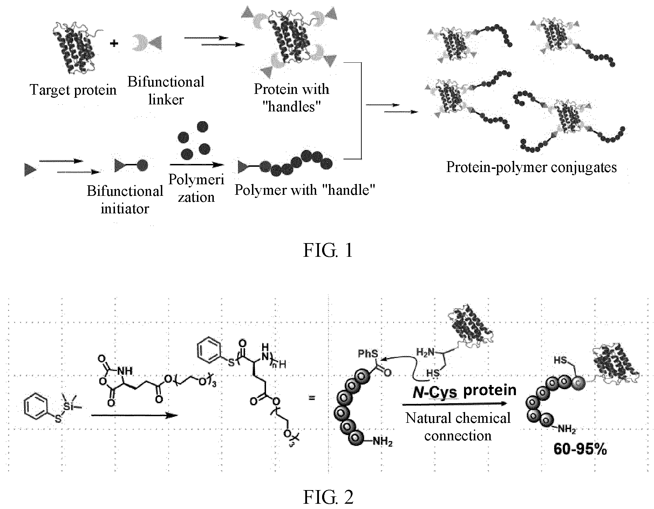

Conjugates of functional molecules such as synthetic proteins (or polypeptides, nucleic acids, drugs, dyes, probes) and polyamino acids have a variety of pharmaceutical and practical functions. However, the synthesis process is usually cumbersome. For example, polyamino acids usually need to be synthesized in advance and then covalently coupled to the target molecule. This strategy is called grafting-to. This strategy has low reaction efficiency and complicated separation and purification. The intermediate process involves tedious steps such as protection, deprotection, or functional group conversion, which are cumbersome and inefficient (Figure 1). Polyamino acids are mainly prepared through the ring-opening polymerization of amino acid N-carboxyanhydrides (also known as NCA). Traditional NCA polymerization uses primary or secondary amines as initiators, uses anhydrous organic solvents such as N,N-dimethylformamide (DMF), acetonitrile (ACN) or tetrahydrofuran (THF) as solvents under strict nitrogen protection, and takes a long time. In addition to the N-terminal amino group, proteins also have a large number of lysine containing pendant amino groups, making proteins useful as an initiator. Therefore, there is a need in the art for new methods of synthesizing polyamino acids and their conjugates.

### SUMMARY OF THE INVENTION

In a long-term research, the inventors achieved controllable polymerization of amino acid N-carboxyanhydride (NCA) in an aqueous phase, water or a mixed solvent of water and an organic solvent. The inventors also performed controllable polymerization of NCA initiated by protein in situ in water or a mixed solvent of water and an organic solvent, thereby preparing protein-poly amino acid conjugates in one step, greatly improving synthesis efficiency. Controllable polymerization as used herein refers to controlling the degree of polymerization or molecular weight by adjusting the ratio of monomers to initiators. The method of the present disclosure achieves coupling reactions within minutes, which is unexpected.

In one aspect, the present disclosure provides a method for polymerizing amino acid N-carboxyanhydrides, which includes adding one or more amino acid N-carboxyanhydrides in water or a mixed solution of water and an organic solvent in the presence of an initiator to react to produce an initiator-polyamino acid conjugate, wherein the organic solvent is selected from the group consisting of: acetonitrile, pyridine, N,N-dimethylformamide, tetrahydrofuran or dimethyl sulfoxide; preferably, the degree of polymerization and molecular weight of the initiator-polyamino acid conjugate are controlled by adjusting the ratio of the initiator and the amino acid N-carboxyanhydride monomer. The degree of polymerization can be greater than 1, such as 25, 50, 60, 70, 90, 90, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 2500, 3000, 3500, 4000, 4500, 5000, 5500, 6000, 6500, 7000, 7500, 8000, 9000 or 10000 or more.

In one embodiment, controllable polymerization can be achieved by adjusting the ratio of initiator and amino acid N-carboxyanhydride monomer. For example, the degree of polymerization can be increased by increasing the molar ratio of amino acid N-carboxyanhydride monomer/initiator. In one embodiment, polyproline is synthesized with a degree of polymerization of 50, 100, 200 or more with a narrow dispersion degree (e.g., 1-1.5) using proline N-carboxyanhydride as a monomer and benzylamine as an initiator.

In one embodiment, the aqueous phase is water or a mixed solution of water and an organic solvent selected from the group consisting of acetonitrile, pyridine, N,N-dimethylformamide, tetrahydrofuran and dimethyl sulfoxide.

In one embodiment, the water content is 20-100 v/v%, for example 30 v/v%, 40 v/v%, 50 v/v%, 60 v/v%, 70 v/v%, 80 v/v%, or 90 v/v%. For example, a mixed solution of water and an organic solvent may be composed of organic solvent and water. The organic solvent may be one of the above-mentioned organic solvents, such as acetonitrile. In one embodiment, the mixed solution of water and an organic solvent may be a mixed solution composed of 0 v/v%-80 v/v% acetonitrile and water. For example, the acetonitrile content is 10 v/v%, 20 v/v%, 30 v/v%, 40 v/v%, 50 v/v%, 60 v/v% or 70 v/v%. In one embodiment, the initiator is a small or large molecule containing a nucleophilic group selected from the group consisting of amino, imino, guanidyl, hydroxyl and thiol.

In one embodiment, the large molecule is a protein, polypeptide or nucleic acid.

In one embodiment, the protein is selected from the group consisting of enzymes, antibodies, cytokines and marker proteins, such as fluorescent proteins, asparaginase, esterase, azoreductase, uricase, dihydrofolate reductase, phenylalanine ammonia lyase (PAL), cystathionine β-synthase (CBS) or hyaluronidase. In one embodiment, the small molecule is a small molecule drug, probe or dye, preferably wherein the small molecule drug is doxorubicin or gemcitabine.

In one embodiment, the reaction is performed at -20°C to 65°C and/or pH 5-9.

In one embodiment, the water or the mixed solution of water and an organic solvent has a salt, such as a buffer salt, preferably one or more selected from the group consisting of sodium salt, potassium salt, magnesium salt and ammonium salt. For example, the aqueous phase can be PBS buffer.

In one embodiment, the amino acids may be selected from natural amino acids and unnatural amino acids. In one embodiment, the amino acids comprise glycine, alanine, valine, leucine, isoleucine, methionine, proline, tryptophan, serine, tyrosine, cysteine, phenylalanine, asparagine, glutamine, threonine, aspartic acid, sarcosine, glutamic acid, lysine, arginine, histidine, ε-nitrogen-benzyloxycarbonyl lysine, glutamic acid benzyl ester, hydroxyproline, (S)-2-amino-4-((3-(benzylamino)-3-oxypropyl)seleno)butyric acid, penicillamine, oxy-tert-butyl-serine, ε-nitrogen-trifluoroacetyl L-lysine, or homocysteine or derivatives of these amino acids, for example, derivatives in which the sulfhydryl group in homocysteine is replaced by a selenoalkoxy group, preferably wherein the amino acid is selected from methionine, valine, leucine, isoleucine, proline, hydroxyproline and alanine or derivatives of these amino acids. In one embodiment, the polyamino acid is a homopolymer or random copolymer of the above amino acids, such as proline, hydroxyproline, serine, threonine, glutamic acid, aspartic acid, glycine, cysteine, methionine, sarcosine, lysine, arginine or derivatives of these amino acids, or random copolymers of these amino acids and other amino acids.

In one embodiment, the concentration of the amino acid N-carboxyanhydride is between 70 and 2800 mM. For example, 70 - 2800 mM proline NCA may be used in the methods of the present disclosure.

In one embodiment, the small molecule is selected from benzylamine, ethylenediamine, p-methylaniline, lysine, glucosamine, p-methylthiophenol, thiol, doxorubicin, and gemcitabine.

In one embodiment, the amino acid N-carboxyanhydride is hydroxyproline N-carboxyanhydride, proline N-carboxyanhydride, glycine N-carboxyanhydride, alanine N-carboxyanhydride, or serine N-carboxyanhydride.

In another aspect, the present disclosure provides a method of producing a protein-polyamino acid conjugate, comprising adding one or more amino acid N-carboxyanhydrides in water or a mixed solution of water and an organic solvent in the presence of a p

rotein to react to produce a protein-polyamino acid conjugate, wherein the organic solvent is selected from the group consisting of acetonitrile, pyridine, N,N-dimethylformamide, tetrahydrofuran, and dimethyl sulfoxide. The method disclosed in the present disclosure initiates a controllable polymerization of NCA in situ by protein in water or a mixed solvent of water and an organic solvent, thereby preparing a protein-polyamino acid conjugate in one step, greatly improving the synthesis efficiency.

In one embodiment, the water content is 20-100 v/v%, for example 30 v/v%, 40 v/v%, 50 v/v%, 60 v/v%, 70 v/v%, 80 v/v%, or 90 v/v%. In one embodiment, the reaction is carried out at -20°C to 65°C and/or pH 5-9.

In one embodiment, the water or the mixed solution of water and an organic solvent has a salt, such as a buffer salt, preferably one or more selected from the group consisting of sodium salt, potassium salt, magnesium salt and ammonium salt.

In one embodiment, the amino acid is selected from natural amino acids and unnatural amino acids. In one embodiment, the amino acids comprise glycine, alanine, valine, leucine, isoleucine, methionine, proline, tryptophan, serine, tyrosine, cysteine, phenylalanine, asparagine, glutamine, threonine, aspartic acid, sarcosine, glutamic acid, lysine, arginine, histidine, ε-nitrogen-benzyloxycarbonyl lysine, glutamic acid benzyl ester, hydroxyproline, (S)-2-amino-4-((3-(benzylamino)-3-oxypropyl)seleno)butyric acid, penicillamine, oxy-tert-butyl-serine, ε-nitrogen-trifluoroacetyl L-lysine, or homocysteine or derivatives of these amino acids, for example, derivatives in which the sulfhydryl group in homocysteine is replaced by a selenoalkoxy group, preferably wherein the amino acid is selected from methionine, valine, leucine, isoleucine, proline, hydroxyproline and alanine or derivatives of these amino acids. In one embodiment, the polyamino acid is a homopolymer or random copolymer of the above amino acids, such as proline, hydroxyproline, serine, threonine, glutamic acid, aspartic acid, glycine, cysteine, methionine, sarcosine, lysine, arginine or derivatives of these amino acids, or random copolymers of these amino acids and other amino acids.

In one embodiment, the amino acid is proline or a derivative thereof, homocysteine or a derivative thereof, glutamic acid or a derivative thereof, e.g., derivatives in which the sulfhydryl group in homocysteine is substituted by a selenoalkoxyalkyl group. In one embodiment, the selenoalkoxyalkyl group is -Se-(CH2CH2O)n-alkyl; wherein n is preferably an integer of 2 or more, such as 3 or 4. In one embodiment, a derivative in which the hydrogen in the hydroxyl group of a glutamic acid side chain is substituted with an alkoxyalkyl group. In one embodiment, the alkoxyalkyl group is (CH2CH2O)n-alkyl; wherein n is preferably an integer of 2 or more, such as 3 or 4. In one embodiment, the proline derivative is: 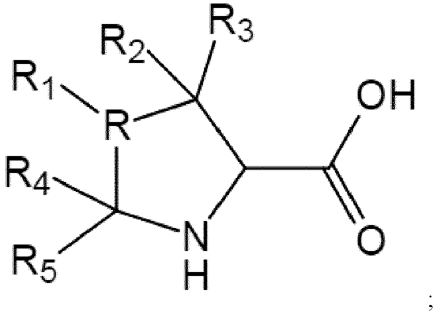
wherein R is CH, N, O, S or Se; R₁ is absent, hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl;
R₂, R₃, R₄ and R₅ are each independently hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, optionally substituted C₆-C₁₂ aryl, or optionally substituted C₆-C₁₂ heteroaryl.

In one embodiment, the proline derivative is: 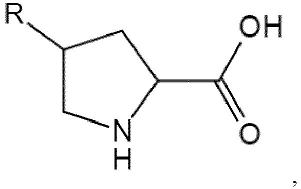
wherein R is hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C-S-C(=O)-optionally substituted C₁₋₆ alkyl, -S-C-S-C(=O)-optionally substituted C₂₋₆ alkenyl, -S-C-S-C(=O)-optionally substituted C₂₋₆ alkynyl, -S-C-S-C(=O)-optionally substituted C₁₋₆ heteroalkyl, -S-C-S-C(=O)-C₂₋₆ heteroalkenyl, -S-C-S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl;
or

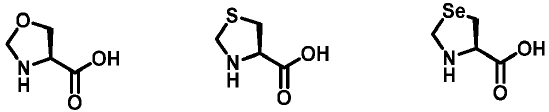

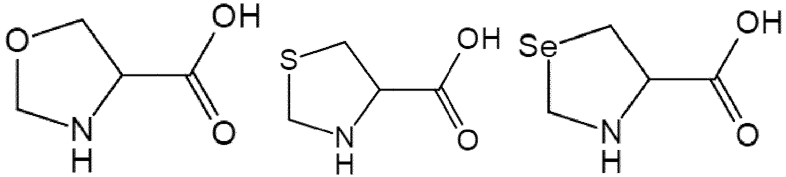

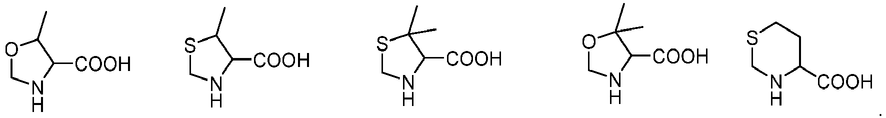

In one embodiment, the protein is selected from enzymes, antibodies, cytokines and marker proteins, such as fluorescent proteins. In one embodiment, the protein is green fluorescent protein, asparaginase, esterase, azoreductase, uricase, dihydrofolate reductase, phenylalanine ammonia lyase, cystathionine beta synthase, or hyaluronidase.

In one aspect, there is provided a use of a polyamino acid to extend the circulation time of a protein or polypeptide in the body, reduce the immunogenicity of a protein or polypeptide, serve as an antifreeze for a protein or polypeptide, or increase the solubility of a drug, wherein the polyamino acid is coupled to proteins or drugs by the method of the present disclosure. In one embodiment, the polyamino acid is selected from: polyproline or proline derivatives or random copolymers of ProNCA, AlaNCA and SerNCA. In one embodiment, the protein is selected from the group consisting of enzymes, antibodies, cytokines and marker proteins, such as fluorescent proteins, asparaginase, esterase, azoreductase, uricase, dihydrofolate reductase, phenylalanine ammonia lyase, cystathionine beta synthase or hyaluronidase. In one embodiment, the small molecule is a small molecule drug, probe, or dye. In one embodiment, the small molecule drug is doxorubicin or gemcitabine. In one embodiment, the proline derivative is: 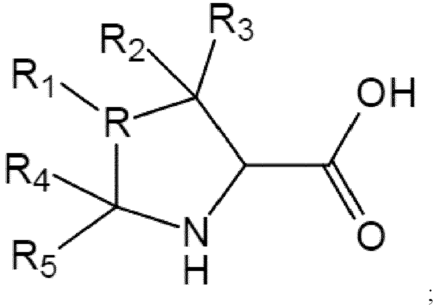
wherein R is CH, N, O, S or Se; R₁ is absent, hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C-S-C(=O)-optionally substituted C₁₋₆ alkyl, -S-C-S-C(=O)-optionally substituted C₂₋₆ alkenyl, -S-C-S-C(=O)-optionally substituted C₂₋₆ alkynyl, -S-C-S-C(=O)-optionally substituted C₁₋₆ heteroalkyl, -S-C-S-C(=O)-C₂₋₆ heteroalkenyl, -S-C-S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl;
R₂, R₃, R₄ and R₅ are each independently hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, optionally substituted C₆-C₁₂ aryl, or optionally substituted C₆-C₁₂ heteroaryl.

In one embodiment, the proline derivative is: 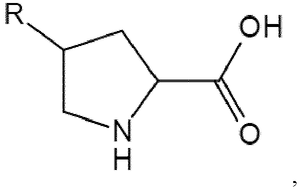
wherein R is hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C-S-C(=O)-optionally substituted C₁₋₆ alkyl, -S-C-S-C(=O)-optionally substituted C₂₋₆ alkenyl, -S-C-S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl;
or

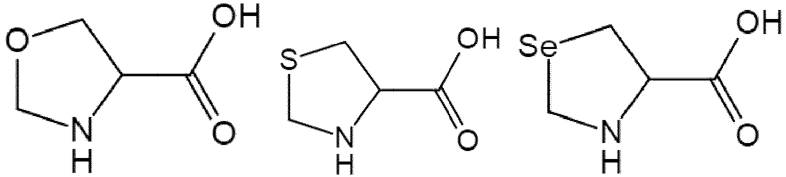

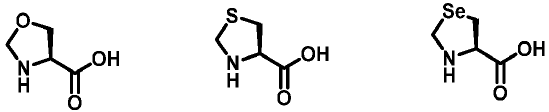

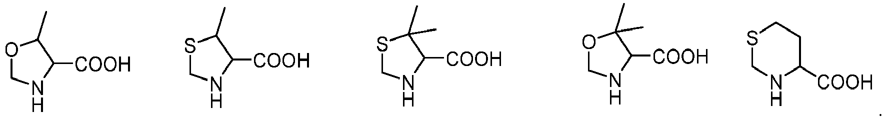

In one aspect, there is provided a conjugate produced by the method of the present application, wherein the initiator is a small molecule or large molecule containing a nucleophilic group, wherein the nucleophilic group is selected from amino, imino, guanidyl, hydroxyl and sulfhydryl. In one embodiment, the large molecule is a protein, polypeptide or nucleic acid. In one embodiment, the protein is selected from the group consisting of enzymes, antibodies, cytokines and marker proteins, such as fluorescent proteins, asparaginase, esterase, azoreductase, uricase, dihydrofolate reductase, phenylalanine ammonia lyase, cystathionine beta synthase or hyaluronidase. In one embodiment, the small molecule is a small molecule drug, probe, or dye. In one embodiment, the small molecule drug is doxorubicin or gemcitabine.

In one aspect, there is provided a conjugate comprising the following formula: 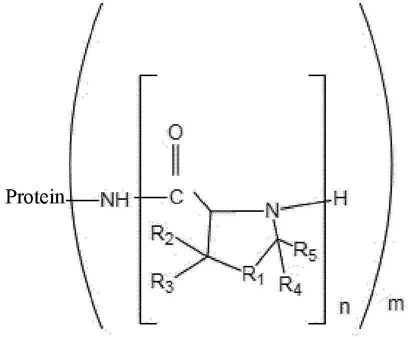 wherein R₁ is NH, CH₂, S, O or Se; R₂, R₃, R₄ and R₅ are each independently hydrogen, optionally substituted C₁-C₁₂ alkyl, optionally substituted C₂₋₆ alkenyl, optionally substituted C₂₋₆ alkynyl, optionally substituted C₁-C₁₂ heteroalkyl, C₂₋₆ heteroalkenyl, C₂₋₆ heteroalkynyl, halogen, hydroxyl, C₆-C₁₂ aryl, C₆-C₁₂ heteroaryl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₁R₂, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₁ and R₂ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl; the protein is selected from enzymes, antibodies, cytokines and marker proteins; wherein n is 1-200, and m is 1-X, wherein X is the number of lysines on the protein that capable for reaction. In one embodiment, the proteins are asparaginase and uricase. In one embodiment, n is 5, 10, 20, 25, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 500, 600, 700, 800, 900, 950, 1000, 1100, 1200, 1300, 1400, 1500, 1600, 1700, 1800, 1900, 2000, 2500, 3000, 3500, 4000, 4500, 5000, 5500, 6000, 6500, 7000, 7500, 8000, 9000 or 10000 or more. In one embodiment, m is 5, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 500, 600, 700, 800, 900, 950, 1000, 1100, 1200, 1300, 1400, 1500, 1600, 1700, 1800, 1900, 2000, 2500, 3000, 3500, 4000, 4500, 5000, 5500, 6000, 6500, 7000, 7500, 8000, 9000 or 10,000 or more. In one embodiment, the polymer has a dispersion degree of from 1 to 1.5, such as 1.03, 1.1, 1.2, 1.3, 1.35, 1.4 or 1.45. In one embodiment, the polymer has a molecular weight of 2.5, 5.0, 10.3 or 18.7 kg/mol.

In one embodiment, for asparaginase-polyproline, m can be 1-160, such as 5, 10, 20, 25, 30, 40, 50, 60, 70, 76, 80, 90, 100, 110, 120, 130, 140 or 150. In one embodiment, for uricase-polyproline, m can be 1-160, such as 5, 10, 20, 25, 30, 40, 50, 60, 70, 80, 90, 100, 110, 120, 128 or 130, 140 or 150.

In one embodiment, the alkyl group is C₁-C₁₂ alkyl, such as C₁-C₆ alkyl, such as methyl, ethyl, propyl, isopropyl, or butyl. In one embodiment, the heteroalkyl is alkoxy or azaalkyl.

In yet another aspect, the present application provides a pharmaceutical composition comprising a conjugate of the present application and a pharmaceutically acceptable carrier.

In yet another aspect, the present application provides use of the conjugate or pharmaceutical composition in the manufacture of a medicament, wherein the protein is asparaginase.

In one embodiment, the drug is used to treat cancer, such as lymphoma or leukemia, or to treat hyperuricemia.

Also provided is a method of treating disease, comprising administering a conjugate or pharmaceutical composition of the present application to a subject in need thereof. In one embodiment, the disease is cancer, such as lymphoma or leukemia.

Advantages of this application include:
1. An in-situ functionalization strategy for polyamino acids is provided, achieving simple and efficient site-directed protein coupling;
2. The method of this application can realize the controllable polymerization of NCA initiated in-situ by protein in water or a mixed solvent of water and an organic solvent;
3. There is provided a small molecule or large molecule conjugate with improved properties, such as improved in vivo half-life, enhanced antifreeze effect, reduced immunogenicity, etc.

### BRIEF DESCRIPTION OF THE DRAWINGS

Figure 1: Traditional Grafting-to protocol for synthesis of protein-polymer conjugates.
Figure 2: Protein-polyamino acid grafting-to coupling method developed by the inventor in the past.
Figure 3: A protein initiates NCA polymerization (grafting-from) in situ in an aqueous phase to synthesize a protein-polyamino acid conjugate.

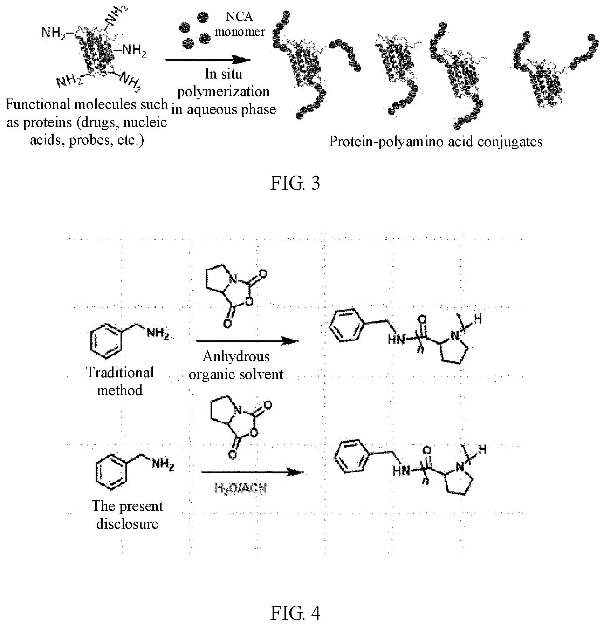

Figure 4: Comparison of a traditional method and a method of the present disclosure. The synthesis reaction of the traditional method is slow and usually takes several days to complete. The yield is low (less than 40%), the molecular weight is low, and the polymerization is uncontrollable (for example, the dispersion degree is greater than 1.7). In comparison, the synthesis reaction of the method disclosed herein is extremely fast and is completed in about 2 minutes. The yield is high (75%-92%), the molecular weight is high, the degree of polymerization can reach 200, and the polymerization is controllable, for example, the dispersion degree is 1.03-1.35.
Figure 5: Comparison of the polymerization speed of the technology disclosed herein and the speed of the traditional method. In the left picture, the traditional method achieves 40 % conversion in 28 hours; in the right picture, the method disclosed herein achieves almost complete conversion in 120 seconds.
Figure 6: Using proline NCA as a monomer and benzylamine as an initiator, the effect of temperature on the polymerization reaction is tested. The polymerization results show a single peak in a range of 0-65 degrees Celsius. The reaction system is turbid above 65 degrees Celsius, but the mixed solution will not freeze below 0 degrees Celsius. It is believed that the polymerization reaction can be carried out between -20 and 65 degrees Celsius.
Figure 7: Using proline NCA as a monomer and benzylamine as an initiator, the effect of pH on the polymerization reaction is tested. pH has almost no effect on the polymerization reaction. pH 5-9 all show a narrow single peak. It is believed that the polymerization reaction can all be carried out at pH 1-14.
Figure 8: Using proline NCA as a monomer and benzylamine as an initiator, the effect of ionic strength on the polymerization reaction is tested. Different sodium chloride concentrations have little effect on the polymerization reaction, wherein 0 mM - 2 M all show a narrow single peak. It is believed that the polymerization reaction can all be carried out at any salt concentration.
Figure 9: Using proline NCA as a monomer and benzylamine as an initiator, the effect of monomer concentration on the polymerization reaction is tested. This figure shows the effect of different monomer concentrations on polymerization when the ratio of monomer to initiator is 200/1. The higher the monomer concentration, the larger the molecular weight and the narrower the distribution.
Figure 10: Using proline NCA as a monomer and benzylamine as an initiator, the effect of different water-organic solvents on the polymerization reaction is tested. The polymerization effects from superior to good are those of ACN, THF, DMSO, DMF, and pyridine in order. The effects of EA, DCM, and CHCl₃ that are immiscible with water are poor.
Figure 11: Using proline NCA as a monomer and benzylamine as an initiator, the effect of the proportion of water in the water-acetonitrile solution on the polymerization reaction is tested. The figure shows a SEC curve obtained by quenching the reaction at 120 seconds. Although the polymerization speed slows down slightly as the water content increases, 20-100 % of water all result in a narrow single peak.
Figure 12: Mass spectrum of the polymerization reaction tested using proline NCA as a monomer and benzylamine as an initiator.
Figure 13: Mass spectrum of the polymerization reaction tested using proline NCA as a monomer and p-methylaniline as an initiator.
Figure 14: Mass spectrum of the polymerization reaction tested using proline NCA as a monomer and diethylamine as an initiator.
Figure 15: Mass spectrum of the polymerization reaction tested using proline NCA as a monomer and tert-butoxycarbonyl-lysine as an initiator.
Figure 16: Mass spectrum of the polymerization reaction tested using proline NCA as a monomer and glucosamine as an initiator.
Figure 17: Mass spectrum of the polymerization reaction tested using proline NCA as a monomer and p-methylthiophenol as an initiator.
Figure 18: Mass spectrum of the polymerization reaction tested using proline NCA as a monomer and mercaptoethanol as an initiator.
Figure 19: Size exclusion chromatogram of the polymerization reaction tested using proline NCA as a monomer and doxorubicin as an initiator.
Figure 20: Size exclusion chromatogram of the polymerization reaction tested using proline NCA as a monomer and gemcitabine as an initiator.
Figure 21: Size exclusion chromatogram (SEC) of L-polyhydroxyproline.
Figure 22: Proton NMR spectrum of copolymers of HypNCA, ProNCA and GlyNCA.
Figure 23: Spectrum of ProNCA, AlaNCA and SerNCA copolymers.
Figure 24: Aqueous phase SEC diagram of ProNCA, AlaNCA and SerNCA copolymers.
Figure 25: SDS-PAGE and Native-PAGE images of L-polyproline-green fluorescent protein conjugate. Polyproline-green fluorescent protein conjugates of different molecular weights can be obtained by polymerization of proline NCA initiated in-situ by EGFP at different monomer concentrations.
Figure 26: SDS-PAGE image of L-polyhydroxyproline-green fluorescent protein conjugate. Polyhydroxyproline-green fluorescent protein conjugates of different molecular weights can be obtained by polymerization of hydroxyproline NCA initiated in-situ by EGFP at different monomer concentrations.
Figure 27: SDS-PAGE image of L-polyproline-dihydrofolate reductase conjugate. Polyproline-dihydrofolate reductase conjugates of different molecular weights can be obtained by polymerization of proline NCA initiated in-situ by DHFR at different monomer concentrations.
Figure 28: Enzyme activity test of polyproline-DHFR conjugates with different molecular weights. The enzyme activity of polyproline-dihydrofolate reductase conjugates with different molecular weights is well retained.
Figure 29: Polymerization of selenium-containing NCA EG₃-SeHC NCA is initiated in situ by EGFP at different pHs.
Figure 30: Polymerization of selenium-containing NCA EG₄-SeHC NCA is initiated in situ by EGFP at different pHs.
Figure 31: Polymerization of EG₃GluNCA is initiated in situ by EGFP at different temperatures.
Figure 32: Polymerization of EG₃GluNCA is initiated in situ by EGFP at different solvents.
Figure 33: Denaturing polyacrylamide gel chromatography (SDS-PAGE) of wild-type asparaginase and asparaginase-polyproline conjugate.
Figure 34: Asparaginase-polyproline conjugate has longer circulation time than wild-type asparaginase.
Figure 35: Asparaginase-polyproline conjugate has lower immunogenicity than wild-type asparaginase.
Figure 36: Asparaginase-polyproline conjugate has better antifreeze effect than commercially available Pegaspargase. Comparison of changes in enzyme activity before and after frozen storage at -20°C.
Figure 37: Asparaginase-polyproline conjugate has better antifreeze effect than commercially available Pegaspargase. Changes in enzyme activity before and after preparation of lyophilized powder.
Figure 38: Comparison of the solubility of doxorubicin hydrochloride and doxorubicin-polyproline conjugate (DOX-PLP).
Figure 39 shows the changes in cell viability as a function of drug concentration.
Figure 40 shows the changes in tumor volume as a function of days of treatment.
Figure 41 shows pictures of histopathological examination.
Figure 42 shows (a) antibody titers of anti-ASNase IgG in different administration groups at week 4; (b) changes in anti-polymer IgG levels at different times; and (c) changes in anti-polymer IgM levels at different times.
Figure 43 shows denaturing polyacrylamide gel chromatogram (left) and circular dichroism spectrum (right).
Figure 44 shows a comparison of the enzyme activities of wild-type uricase and uricase-polyproline conjugate (UOx-PLP).
Figure 45 shows a comparison of the unfolding temperatures of wild-type uricase and uricase-polyproline conjugate (UOx-PLP).
Figure 46 shows a comparison of enzyme activity retention of wild-type uricase and uricase-polyproline conjugate (UOx-PLP) under thermal stimulation.
Figure 47 shows the in vivo half-life of wild-type uricase and uricase-polyproline conj ugate.
Figure 48 shows a comparison of the in vivo immunogenicity of wild-type uricase and uricase-polyproline conjugate.
Figure 49 shows the in vivo safety evaluation of wild-type uricase and uricase-polyproline conjugate.

### DETAILED DESCRIPTION OF THE INVENTION

As used herein, "amino acid" may refer to a compound having a structure H₂N-R^{x}-COOH, wherein R^{x} is an organic group, and wherein NH₂ may optionally be combined with R^{x} (e.g., as in the case of proline). Amino acid derivatives refer to compounds obtained by substituting amino acid atoms. Amino acids include any known amino acids, including but not limited to alpha amino acids, beta amino acids, gamma amino acids, delta amino acids, etc. In some embodiments, the term may refer to an alpha or beta amino acid. In the present disclosure, the amino acid can be 20 natural amino acids, such as glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine or histidine. Alternatively, amino acid derivatives refer to compounds obtained by substituting amino acid atoms, such as ε-nitrogen-benzyloxycarbonyllysine, glutamic acid benzyl ester, sarcosine, hydroxyproline, (S)-2-amino -4-((3-(benzylamino)-3-oxypropyl)seleno)butyric acid, γ-(2-(2-(2-(2-methoxyethoxy)ethoxy)glutamic acid, penicillamine, phenyllactic acid, mandelic acid, or oxy-tert-butyl-serine, or amino acids protected by a protecting group such as Boc or Cbz, such as various naturally occurring amino acids protected by Boc or Cbz. The protection of protecting groups can be the protection of α-amino group, hydroxyl group, thiol group, carboxyl group, or the protection of the corresponding group on the side chain, or both at the same time. The chirality of the above-mentioned amino acids is not limited and can be L-amino acid or D-amino acids. In this application, an amino acid structure represented by a chirality represents L-amino acid or D-amino acid where applicable. According to one embodiment of the application, the amino acid is a natural amino acid or an unnatural amino acid. The unnatural amino acid can be the above-mentioned natural amino acid that has been artificially modified, for example, tyrosine, serine, threonine, cysteine, aspartic acid and/or glutamic acid that are modified by oligoethylene glycol (the degree of polymerization is 2-10, such as triethylene glycol, EG3), phosphate, propyleneoxy benzyl ester, or allyl triethylene glycol, or tyrosine, serine, threonine, cysteine, aspartic acid and/or glutamic acid that are modified by triethylene glycol (EG3).

Amino acid N-carboxyanhydride can be represented by formula (I): 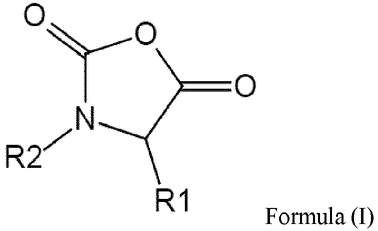
wherein R₁ is -R₄-R₅-R₅, wherein R₄ is H, optionally substituted C₁₋₆ linear or branched alkyl (for example, C₁₋₆ linear or branched alkyl substituted by hydroxyl or mercapto), optionally substituted C₁₋₆ alkoxy, carboxyl, amino, carbonylamino or aminocarbonyl, guanidyl, optionally substituted phenyl (e.g., substituted by hydroxyl), optionally substituted benzyl (e.g., substituted by hydroxyl), indolyl, imidazolyl, guanidyl, or carbonyl alkoxy, or alkoxycarbonyl;
R₅ is absent, oxygen group, selenium group, thio group, carbonyl group, ester group, ester imino or imino ester group, imino, amino, carbonylamino, carbonylimino or iminocarbonyl group, benzyl ester group, optionally substituted phenyl (for example, substituted by hydroxyl), optionally substituted benzyl (for example, substituted by hydroxyl), indolyl, imidazolyl, guanidyl, carbonylalkoxy or alkoxycarbonyl, hydroxyl, mercapto, or carboxyl;
R₆ is absent, H, optionally substituted C₁₋₆ linear or branched alkyl (for example, C₁₋₆ linear or branched alkyl substituted by hydroxyl, mercapto or halogen); optionally substituted C₁₋₆ alkoxy group, hydroxyl group, carboxyl group, mercapto group, amino group, carbonylamino group, guanidyl group, optionally substituted phenyl group (for example, substituted by hydroxyl group), amino protecting group, hydroxyl protecting group or carboxyl protecting group, optionally substituted benzyl (e.g. substituted by hydroxy), optionally substituted phenylalkyl, optionally substituted benzylalkyl, indolyl, imidazolyl, guanidyl, carbonylalkoxy or alkoxycarbonyl, benzyl ester group, benzyliminocarbonylalkyl group, trifluoroacetyl group, tert-butoxycarbonyl group or -[O(CH₂)ₘ₁]ₘ₂-O-R₇,
wherein R₇ is H, optionally substituted C₁₋₆ linear or branched alkyl (for example, C₁₋₆ linear or branched alkyl substituted by hydroxyl or mercapto), optionally substituted C₁₋₆ alkoxy, carbonylalkyl, or iminoalkyl; m1 and m2 are each independently an integer from 1 to 6;
R₂ represents absence, H, halogen, optionally substituted C₁₋₆ linear or branched alkyl (for example, C₁₋₆ linear or branched alkyl substituted by hydroxyl or mercapto), optionally substituted C₂₋₆ linear or branched alkenyl or alkynyl, C₁₋₆ alkoxy, cycloalkyl, aryl or heterocyclyl, wherein one or more hydrogen atoms are optionally substituted by halogen, oxygen or nitrogen, or amino protecting group such as Boc or Cbz.

The amino acid N-carboxyanhydride can be represented by formula (II): 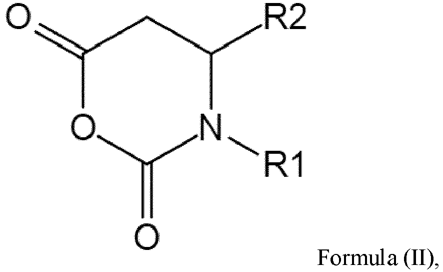 wherein R₁ represents H, halogen, optionally substituted C₁₋₆ linear or branched alkyl, optionally substituted C₂₋₆ linear or branched alkenyl or alkynyl, C₁₋₆ alkoxy, cycloalkyl, aryl or heterocyclyl, such as benzyl, wherein one or more hydrogen atoms are optionally substituted by halogen, oxygen or nitrogen, or Boc or Cbz; R₂ is H, optionally substituted C₁₋₆ linear or branched alkyl (for example, C₁₋₆ linear or branched alkyl substituted by hydroxyl or mercapto) or optionally substituted C₁₋₆ alkoxy or optionally substituted aryl, for example phenyl substituted by halogen, C₁₋₆ linear or branched alkyl group, hydroxyl group, or mercapto group, such as methylphenyl group, or carbonyloxyalkyl group, such as methyl ester group, ethyl ester group, or propyl ester group.

As used herein, the term "initiator" refers to a chemical molecule used to initiate the ring-opening polymerization (ROP) reaction of amino acid N-carboxyanhydride through a nucleophilic mechanism, wherein the initiator is incorporated into the backbone of the resulting polyamino acid. The initiator may contain one or more nucleophilic groups that can initiate the ROP reaction, and thus the initiator may be monofunctional or polyfunctional, respectively, producing one or more polymeric chains.

In one embodiment, the initiator is a small or large molecule containing a nucleophilic group selected from amino, imino, hydroxyl and thiol groups. Large molecules can be nucleic acids or proteins.

According to one embodiment of the present application, the protein is a protein selected from the group consisting of enzymes, hormones, cytokines, monoclonal antibodies and/or protein vaccines. According to another embodiment of the present application, the protein is a protein with medical use, for example, peptide hormones, monoclonal antibodies, interferons, interleukins, colony-stimulating factors, recombinant vaccines, etc. In this embodiment, proteins include various antibodies such as infliximab, adalimumab, rituximab, and other anti-PD-1/PD-L1, anti-CTLA-4, anti-EGFR, anti-HER2, anti-TNFα, anti-CD19, anti-CD33, anti-CD30, anti-CD20, anti-CD25 antibodies; enzymes such as uricase, asparaginase, arginase, carboxypeptide acid, phenylalanine ammonia lyase; growth factors and cytokines, such as growth hormone, G-CSF, cytokines and chemokines (IL-2, interferon-α2a, interferon-α2b, interferon-2a, coagulation factor VIII, coagulation factor IX, interferon-β1a, interferon-γ).

According to one embodiment of the present application, enzymes that can be used in the present application include, but are not limited to: proteolytic enzymes, amylase, lipase, cellulase, trypsin, chymotrypsin, streptokinase, urokinase, plasmin, thrombin, glutaminase, arginase, serine dehydratase, phenylalanine ammonia lyase, leucine dehydrogenase, penicillinase, superoxide dismutase, uricase, glucanase and/or dextranase.

"Asparaginase" or ASNase is an enzyme that can break down asparagine into aspartic acid and ammonia with high selectivity. Asparaginase has a significant effect on leukemia. However, if asparaginase is injected directly into the body, it is easily decomposed by proteases and has a short half-life in the blood. Repeated injections of free asparaginase can produce antibody antigen responses and allergic reactions. Polyethylene glycol (PEG) is a water-soluble polymer with low toxicity and low immune response. A PEG-modified asparaginase, Pegaspargase, has also been reported. Because Pegaspargase has been modified with PEG, it has high substrate specificity and overcomes the immunogenicity and severe allergic reaction activity of asparaginase. The antigenicity of Pegaspargase is lower than that of natural L-asparaginase, and has a longer half-life. However, there remains a need in the art for asparaginase that can be further improved. The inventor found in long-term research that by modifying asparaginase with polyproline or its derivatives, the in vivo half-life, in vivo activity and immunogenicity of asparaginase can be improved by forming an amide bond between the amino group of asparaginase and polyproline. Unexpectedly, the inventor also found that compared with Pegaspargase, the asparaginase-polyproline conjugate prepared in the present application also has better antifreeze effect. The inventor also found that compared with PEG-modified asparaginase, the asparaginase-polyproline conjugate prepared in the present application has lower immunogenicity. In this disclosure, a 500 µL solution of 12 mg/mL L-proline N-carboxyanhydride in acetonitrile (e.g., 500 µL 12 mg/mL) can be mixed and reacted with a solution of asparaginase in PBS (e.g., 500 µL 4.75 mg/mL) (for example, 10 minutes), and the product is purified using a size exclusion column such as Superdex 200 increase 10/300 GL.

Uricase, also known as urate oxidase, can catalyze uric acid to produce allantoin and is an important enzyme in purine metabolism. Lack of uricase will lead to a decrease in the ability to metabolize uric acid, making it susceptible to various diseases caused by the accumulation of uric acid. Therefore, uricase, as a class of therapeutic drugs, has a wide range of clinical applications. The three uricase drugs that have been marketed still have problems such as short half-life and strong immunogenicity. The inventor found that by conjugating uricase with polyproline, the activity of uricase can be retained, the half-life in vivo can be prolonged, and the immunogenicity can be reduced. In this disclosure, a solution of L-proline N-carboxyanhydride in acetonitrile (for example, 500 µL 7.4 mg/mL) can be mixed and reacted with a solution of uricase in PBS (for example, 500 µL 2.0 mg/mL, pH 7.4) (for example, 5 minutes), and the product is purified by a size exclusion chromatography column, such as Superdex 200 increase 10/300 GL.

According to one embodiment of the present application, hormones that can be used in the present application include, but are not limited to, hypothalamic hormones, pituitary hormones, gastrointestinal hormones, insulin, and calcitonin.

According to one embodiment of the present application, cytokines useful in the present application include, but are not limited to, interleukins, interferons, colony-stimulating factors, chemokines and/or growth factors.

According to one embodiment of the present application, interleukins that can be used in the present application include IL-1, IL-2, IL-3, IL-4, IL-5, IL-6, IL-7, IL-8, IL -9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31 and/or IL-32.

According to one embodiment of the present application, interferons that can be used in the present application include, but are not limited to, IFN-α, IFN-β, IFN-γ, IFN-λ and subtypes thereof.

According to one embodiment of the present application, colony-stimulating factors that can be used in the present application include, but are not limited to: granulocyte colony-stimulating factor, macrophage colony-stimulating factor, granulocyte-macrophage colony-stimulating factor, pluripotent colony-stimulating factor, stem cell factor, leukemia inhibitory factor, and/or erythropoietin.

According to one embodiment of the present application, growth factors useful in the present application include, but are not limited to, epidermal cell growth factor, transforming growth factor, insulin-like growth factor and/or nerve growth factor.

According to one embodiment of the present application, monoclonal antibodies that can be used in the present application include, but are not limited to: trastuzumab, cetuximab, daclizumab, tanezumab, abagovomab, adecatumumab, afutuzumab, alemtuzumab, PEG-alacizumab, amatuximab, apolizumab, bavituximab, bectumomab, belimumab, bevacizumab, bivatuzumab mertansine, brentuximab vedotin, cantuzumab mertansin, cantuzumab ravtansine, capromab pendetide, catumaxomab, citatuzumab bogatox, cixutumumab, conatumumab, dacetuzumab, dalotuzumab, detumomab, ecromeximab, edrecolomab, elotuzumab, ensituximab, epratuzumab, ertumaxomab, etaracizumab, farletuzumab, figitumumab, galiximab, gemtuzumab, gemtuzumab, girentuximab, glembatumumab vedotin, ibritumomab tiuxetan, igovomab, indatuximab ravtansine, intetumumab, inotuzumab ozogamicin, ipilimumab, iratumumab, labetuzumab, lexatumumab, lintuzumab, lorvotuzumab mertansine, lucatumumab, lumiliximab, mapatumumab, matuzumab, milatuzumab, mitumomab, mogamulizumab, nacolomab tafenatox, naptumomab estafenatox, necitumumab, nimotuzumab, nivolumab, ofatumumab, omalizumab, oportuzumab monatox, oregovomab, pemtumomab, patritumab, pertuzumab, pritumumab, racotumomab, ramucirumab, rilotumumab, rituximab, robatumumab, omalizumab, sibrotuzumab, siltuximab, taplitumomab paptox, tenatumomab, teprotumumab, ticilimumab, tremelimumab, tigatuzumab, tositumomab, tucotuzumab celmoleukin, urelumab, veltuzumab, volociximab, votumumab and zalutumumab, including antigen binding fragments thereof.

As used herein, "alkyl" refers to a straight or branched chain saturated hydrocarbon having from 1 to 30 carbon atoms, which may be optionally substituted, allowing various degrees of substitution, as further described herein. As used herein, examples of "alkyl" include, but are not limited to, methyl, ethyl, n-propyl, isopropyl, isobutyl, n-butyl, sec-butyl, tert-butyl, isopentyl, n-pentyl, neopentyl, n-hexyl and 2-ethylhexyl. In some cases, one or more carbon atoms in an alkyl group may be substituted with a heteroatom, and the alkyl group is referred to as a "heteroalkyl group." Non-limiting examples include alkoxy, which refers to a group in which a carbon atom in an alkyl group is replaced by oxygen. The alkyl group may be C₁₋₆ alkyl. "C₁₋₆ alkyl" means an alkyl group having 1 to 6 carbon atoms, and includes, for example, but is not limited to, methyl, ethyl, n-propyl, isopropyl, isobutyl, n-butyl, sec-butyl, tert-butyl, isopentyl, n-pentyl, neopentyl and n-hexyl. "C₁₋₆ alkoxy" means a C₁₋₆ alkyl group in which one or more carbon atoms are replaced by oxygen, such as methoxy, ethoxy, n-butoxy, tert-butoxy, pentyloxy or hexyloxy. "Alkyl" or "alkoxy" may also be substituted by halogen, alkyl, hydroxyl, mercapto, carboxyl, aryl, etc. Alkoxy may refer to C₁₋₆ alkoxy.

"Heteroalkyl" as used herein refers to an alkyl group in which one or more (e.g., one to four) carbon atoms are replaced by a group selected from -O-, -S- and -N-. In one embodiment, heteroalkyl is C₁-C₁₂ heteroalkyl, such as C₁-C₆ heteroalkyl.

"Aryl" refers to a ring system containing at least one carbocyclic aromatic ring. In certain embodiments, an aryl group contains 6 to 18 carbon atoms, such as 6 to 12 carbon atoms. Aryl rings may be monocyclic, bicyclic, tricyclic or tetracyclic ring systems, which may include fused or bridged ring systems. Aryl groups include, but are not limited to, aryl groups derived from: aceanthrylene, acenaphthylene, acephenanthrylene, anthracene, azulene, benzene, fluoranthene, fluorene, as-indacene, s-indacene, indane, indene, naphthalene, phenalene, phenanthrene, pleiadene, pyrene, and triphenylene. Unless stated otherwise in the specification, aryl groups are optionally substituted.

"Heteroaryl" refers to a 5- to 14-membered ring system comprising one to thirteen carbon atoms; one to six heteroatoms selected from nitrogen, oxygen, and sulfur; and at least one aromatic ring. For purposes of certain embodiments of the present disclosure, the heteroaryl residue may be a monocyclic, bicyclic, tricyclic, or tetracyclic ring system, which may include fused or bridged ring systems; the nitrogen, carbon or sulfur atoms in the heteroaryl residues may be optionally oxidized; and the nitrogen atoms may be optionally quaternized. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, benzoxazolinonyl, benzimidazolesulfinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1H-pyrrolyl, phenazinyl, phenothiazinyl, phenoxazinyl, phthalazinyl, pteridinyl, pteridinonyl, purinyl, pyrrolyl, pyrazolyl, pyridinyl, pyridinonyl, pyrazinyl, pyrimidinyl, pyrimidinonyl, pyridazinyl, pyrrolyl, pyrido[2,3-d]pyrimidinonyl, quinazolinyl, quinazolinonyl, quinoxalinyl, quinoxalinonyl, quinolinyl, isoquinolinyl, tetrahydroquinolinyl, thiazolyl, thiadiazolyl, thieno[3,2-d]pyrimidin-4-onyl, thieno[2,3-d]pyrimidin-4-onyl, triazolyl, tetrazolyl, triazinyl, and thiophenyl (i.e., thienyl). Unless stated otherwise in the specification, a heteroaryl group is optionally substituted.

As used herein, the term "alkenyl" refers to an alkyl group as defined herein having one or more double bonds. The term "heteroalkenyl" refers to an alkenyl group in which one or more carbon atoms are replaced by a heteroatom (i.e., nitrogen, oxygen, or sulfur, including any oxidized form of nitrogen or sulfur, and any quaternized form of basic nitrogen). In one embodiment, alkenyl is C₂-C₆ alkenyl. In one embodiment, heteroalkenyl is C₂-C₆ heteroalkenyl.

As used herein, the term "alkynyl" refers to an alkyl group as defined herein having one or more triple bonds. As used herein, the term "heteroalkynyl" refers to an alkynyl group as defined herein in which one or more constituent carbon atoms have been replaced by nitrogen, oxygen, or sulfur. In one embodiment, the alkynyl group is C₂-C₆ alkynyl. In one embodiment, the heteroalkynyl group is C₂-C₆ heteroalkynyl.

### Synthesis of polyamino acid-protein

Conjugates of functional molecules such as synthetic proteins (or nucleic acids, drugs, dyes, probes) and polyamino acids have a variety of pharmaceutical and practical functions. However, the synthesis process is usually cumbersome. For example, polyamino acids usually need to be synthesized in advance and then covalently coupled to target molecules. This strategy is called grafting-to. This strategy has low reaction efficiency and complicated separation and purification. The intermediate process involves tedious steps such as protection, deprotection, or functional group conversion, which are cumbersome and inefficient (Figure 1).

The inventor has developed an in-situ functionalization strategy for polyamino acids since 2016, achieving simple and efficient site-directed protein coupling (Figure 2, an overall process of 2-3 steps). However, the above method still falls within the scope of grafting-to. If a controllable polymerization of NCA initiated by protein in situ can be achieved in water or a mixed solvent of water and an organic solvent (i.e., the grafting-from scheme), protein-polyamino acid conjugates can be prepared in one step, greatly improving the synthesis efficiency (Figure 3).

### Synthesis of polyproline-protein

Polyproline is an important water-soluble polymer. Literature has shown that coupling proteins to polyproline has a certain effect in extending protein circulation time (US patent 4,894,226, grafting-to coupling proteins to polyproline). In addition, study has shown that polyproline has a certain anti-icing effect (Angew. Chem., Int. Ed. 2017, 56 (50), 15941-15944.). If polyproline is blended or covalently coupled with protein, the polyproline may improve the stability of the protein during repeated freeze-thaw or freeze-drying processes as an antifreeze or freeze-drying protectant.

However, the synthesis of polyproline is extremely difficult: a solid-phase peptide synthesis method can only obtain very short (polymerization degree<50) oligomers, while the ring-opening polymerization of proline NCA (ProNCA) using the traditional organic phase polymerization method has harsh conditions, poor reaction activity (taking several days and incomplete conversion), and very low yield; in addition, because the polymer obtained has poor solubility in organic solvents, oligomers are generated during polymerization in organic solvents and then precipitate, making it impossible to obtain high polymer.

This disclosure screened a variety of mixed solutions of organic solvents and water, and unexpectedly found that ProNCA can achieve rapid and molecular weight-controlled ring-opening polymerization in mixed solutions of acetonitrile, pyridine, THF, DMF, or DMSO and water. The reaction can be completed within 2 minutes, the degree of polymerization of the obtained polymer can reach 200, the degree of dispersion is narrow, and the yield can reach 75-92 % (**Scheme 1, Table 1**). In one embodiment, a solution of proline N-carboxyanhydride in acetonitrile (e.g., 500 µL 7.4 mg/mL) can be mixed and reacted with a solution of an enzyme in PBS (e.g., 500 µL 2.0 mg/mL, pH 7.4) (e.g., for 5 minutes), and the product is purified, for example, using a size exclusion chromatography column, such as Superdex 200 increase 10/300 GL.

### Example

The following examples show certain illustrative embodiments of compounds, compositions, and methods disclosed herein. These examples should not be considered limiting in any way. Nor should these examples be construed as expressing any preferred embodiments, or as indicating any direction for further research. The experimental methods used in the following examples are conventional methods unless otherwise specified. The materials, reagents, etc. used can be obtained from commercial sources unless otherwise specified.

### Example 1: Comparison of the traditional method and the method of the present disclosure

### Preparation of polyproline by the traditional method

In a 10 °C cold storage, L-proline N-carboxyanhydride (50 mg, 0.35 mmol, 100 equivalent) was added into a 5 mL glass bottle, and 500 µL of analytical anhydrous acetonitrile and benzylamine (0.5 M × 7 µL, 0.0035 mmol, 1 equivalent) were added. The mixture was reacted for several days. The product was passed through a PD 10 desalting column and lyophilized to obtain the final product, with a yield of 48%, a molecular weight of 5k (double peaks), and a PDI of 15 (double peaks).

### Preparation of polyproline by the method of the present disclosure

In a 10 °C cold storage, L-proline N-carboxyanhydride (50 mg, 0.35 mmol, 100 equivalents) and benzylamine (0.5 M × 7 µL, 0.0035 mmol, 1 equivalent) were added into a 5 mL glass bottle. 500 µL of analytical grade acetonitrile and 500 µL of double-distilled water were added and the mixture was reacted for 10 minutes. The product was passed through a PD 10 desalting column and lyophilized to obtain the final product, with a yield of 88%, a molecular weight of 10k, and a PDI of 1.07. Compared with the traditional amino acid polymerization method using an organic solvent, the method disclosed herein has a higher yield of generating polyamino acids, and the degree of polymerization and polymer dispersion index PDI of the obtained polyamino acids were better than those obtained by the traditional method.

In the traditional method and the method of the present disclosure, the conversion rate of proline is calculated by taking samples at different time points. Specifically, for the traditional method, samples are collected at 0, 1, 2, 4, 7, and 28 hours to determine the conversion rate of polyproline, and for the method of the present disclosure, samples are collected at 10, 20, 20, 40, 50, 60, 80, 100, and 120 seconds to determine the conversion rate of proline.
Traditional method: quantification based on infrared absorption at 1853 cm⁻¹; conversion rate = 1-remaining absorption value/initial absorption value;
This disclosure uses NMR for quantification: conversion rate = polymer integral/(polymer integral + amino acid integral)

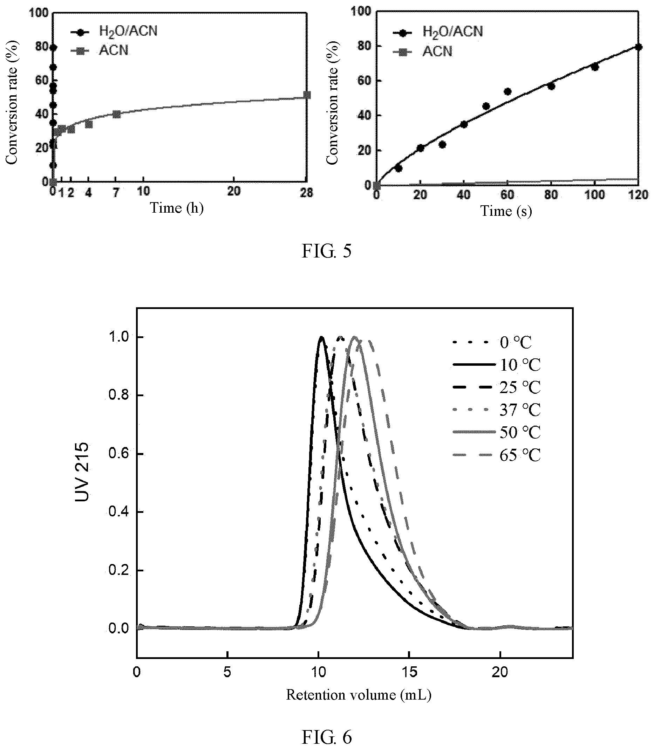

Comparison of the synthesis speed of polyproline prepared by the traditional method and polyproline prepared by the method of the present disclosure, as shown in Figures 4 and 5, the method of the present disclosure can be completed in 2 minutes compared to the reaction that takes several days to complete by the traditional method. Moreover, the yield, degree of polymerization, and degree of dispersion of the method disclosed herein are superior to the traditional method.

### Products obtained with different monomer/initiator molar ratios

In a 10 °C cold storage, L-proline N-carboxyanhydride and benzylamine were added into a 5 mL glass bottle, and 500 µL of analytical grade acetonitrile and 500 µL of double-distilled water were added. The mixture was reacted for 10 minutes. The product was passed through a PD 10 desalting column and lyophilized to obtain the final product. The molar ratio of L-proline N-carboxyanhydride to benzylamine was provided in Table 1, and the polyproline product was synthesized by the method of the present disclosure as described above. The theoretical molecular weight of the obtained product is consistent with the actual molecular weight, and the dispersion degree is narrow. It is shown that the polyproline of the present disclosure can be polymerized in a controllable manner by adjusting the monomer/initiator molar ratio.

**Table 1. Molecular weight and dispersion degree data of polyproline prepared by the present disclosure**

| **The molar ratio of monomer/initiator** | **Theoretical molecular weight (kg/mol)** | **Actual molecular weight (kg/mol)** | **Dispersion degree *Ð*** |
|---|---|---|---|
| 25 | 2.5 | 2.5 | 1.05 |
| 50 | 5.0 | 5.0 | 1.15 |
| 100 | 9.8 | 10.3 | 1.07 |
| 200 | 19.5 | 18.7 | 1.18 |

### Example 2: Screening of reaction conditions

As described in Example 1, in a 10° C cold storage, L-proline N-carboxyanhydride (50 mg, 0.35 mmol, 50 equivalents) and benzylamine (0.5 M × 14 µL, 0.007 mmol, 1 equivalent) were added into a 5 mL glass bottle, and 500 µL of analytical grade acetonitrile and 500 µL of double-distilled water were added. The mixture was reacted for 10 minutes. The product was passed through a PD 10 desalting column and lyophilized to obtain the final product. Superdex75 10/300 GL column combined with AKTA was used for analysis. 100 µL of 0.5 mg/mL polymer was loaded, and the mobile phase was PBS at a flow rate of 1 mL/min.

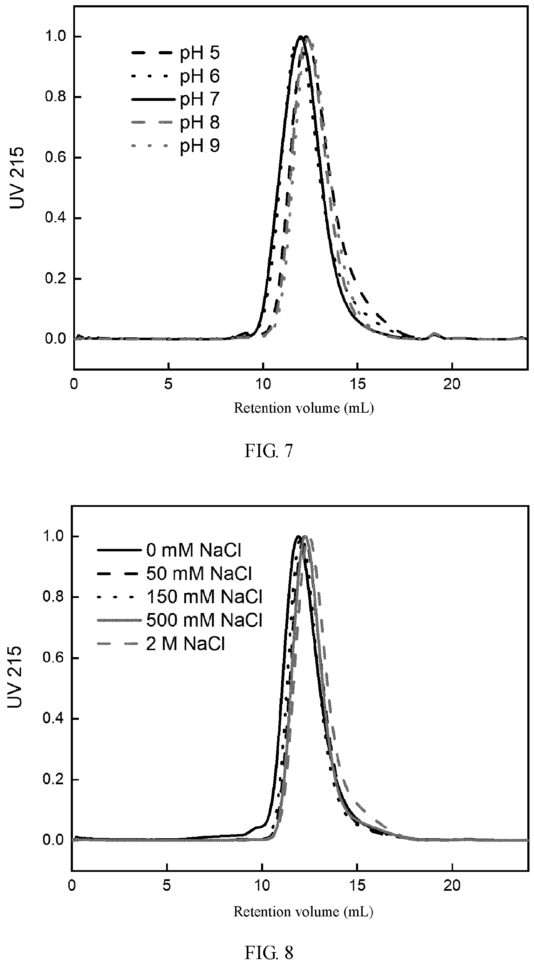

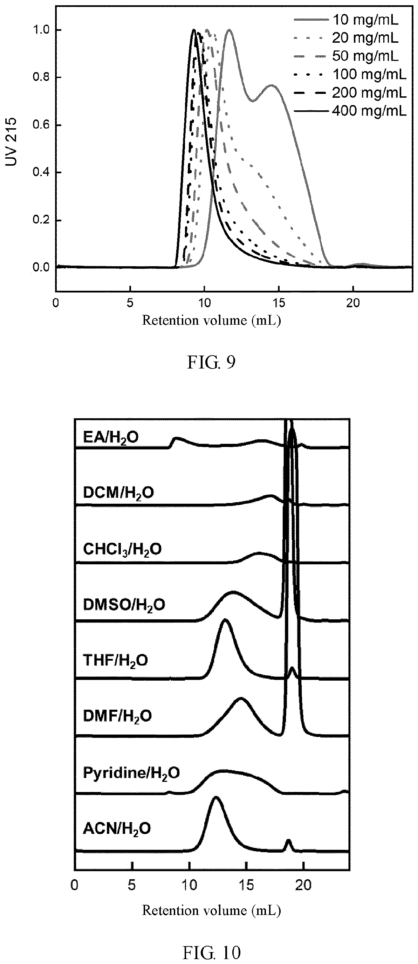

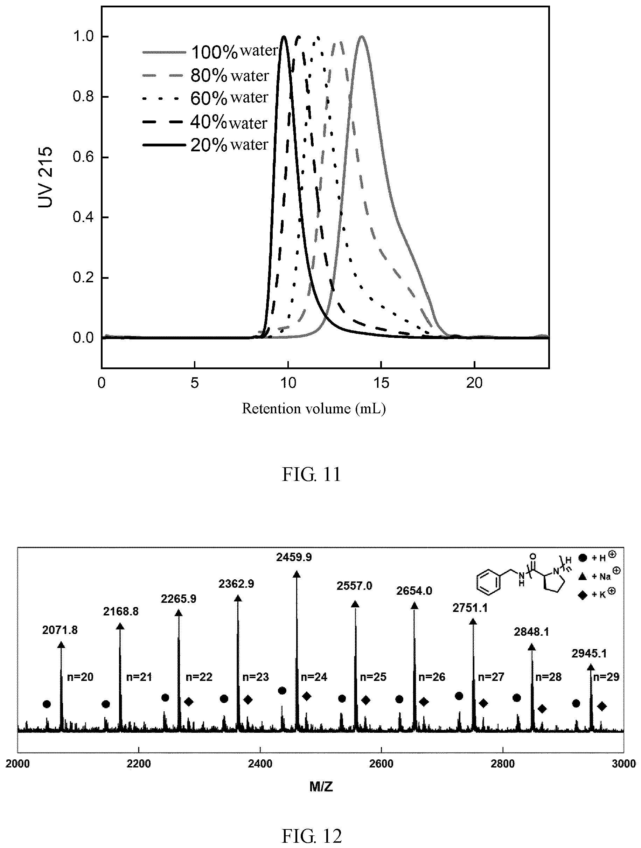

The reaction conditions were screened under a series of conditions, including different temperatures, different pH values, different ionic strengths, different monomer concentrations, different solvents combined with water, and different ratios of water and acetonitrile. The results, shown in Figures 6-11, demonstrate that the methods of the present disclosure can be performed at temperatures of 0-65°C, pH 5-9, various ionic strengths (e.g., 0-2M NaCl) and monomer concentrations, and in various organic solvents such as acetonitrile, pyridine, N,N-dimethylformamide, tetrahydrofuran or dimethyl sulfoxide and various ratios. The polymerization effects from superior to good are those of ACN, THF, DMSO, DMF, and pyridine in order. The effects of EA, DCM, and CHCl₃ that are immiscible with water are poor. Figure 11 shows a SEC curve obtained by quenching the reaction at 120 seconds. Although the polymerization speed slows down slightly as the water content increases, 20-100 % of water all results in narrower single peaks. The inventor also found that the best result was obtained in a 20% acetonitrile aqueous solution compared to other concentrations of acetonitrile aqueous solutions.

### Example 3: Screening of initiators

As described in Example 1, in a 10°C cold storage, L-proline N-carboxyanhydride (50 mg, 0.35 mmol, 50 equivalents) and benzylamine (0.5 M × 14 µL, 0.007 mmol, 1 equivalent) were added into a 5 mL glass bottle, and 500 µL of analytical grade acetonitrile and 500 µL of double-distilled water were added. The mixture was reacted for 10 minutes. The product was passed through a PD 10 desalting column and lyophilized to obtain the final product. Similarly, under the condition that other conditions remained unchanged, ethylenediamine, p-methylaniline, lysine, glucosamine, p-methylthiophenol and mercaptan were used to replace benzylamine as an initiator, and doxorubicin and gemcitabine were used to replace benzylamine as an initiator. Spectra were obtained by MALDI-TOF or, for doxorubicin and gemcitabine, size exclusion chromatograms were obtained.

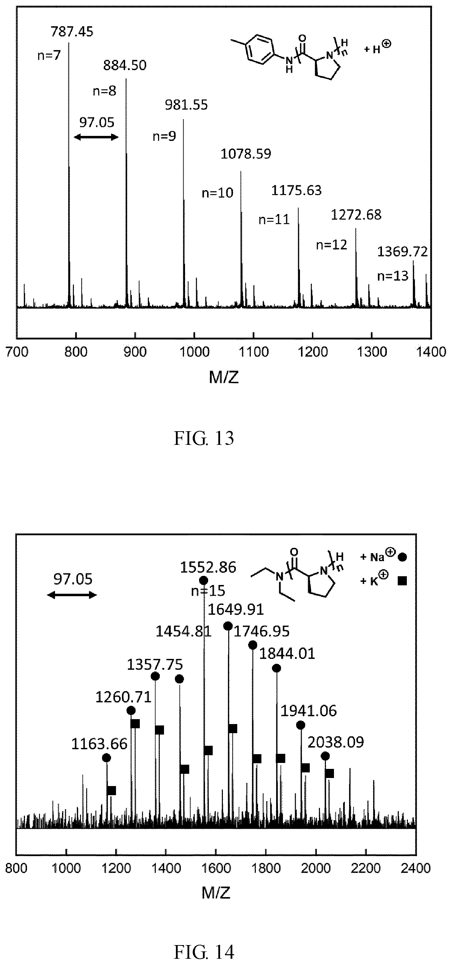

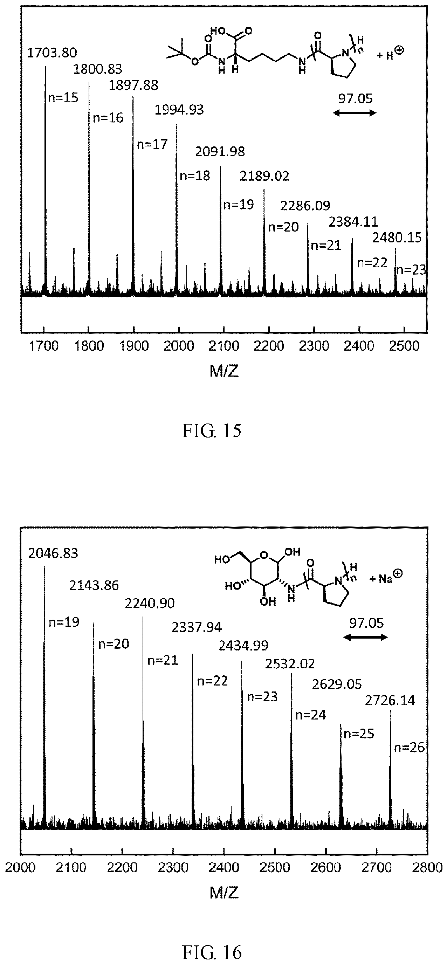

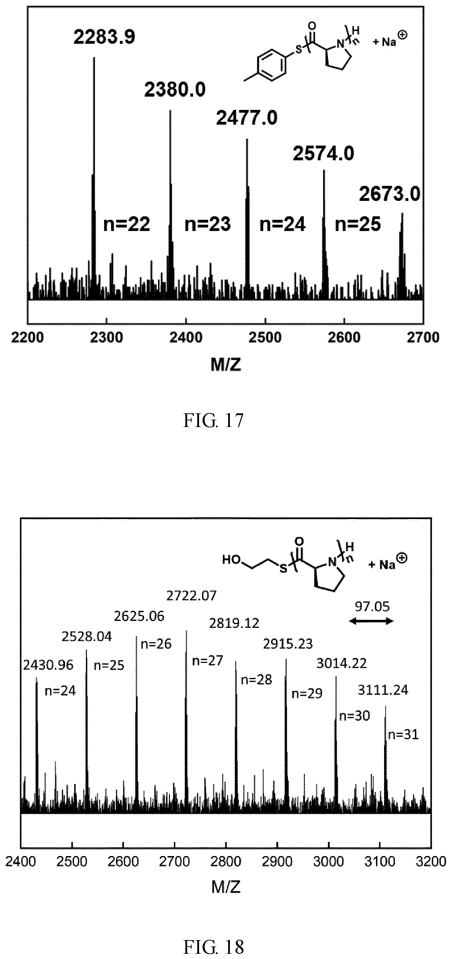

The results in Figures 12-20 show that the synthesis of polyproline can be initiated using different initiators.

### Example 4: Synthesis of polyhydroxyproline.

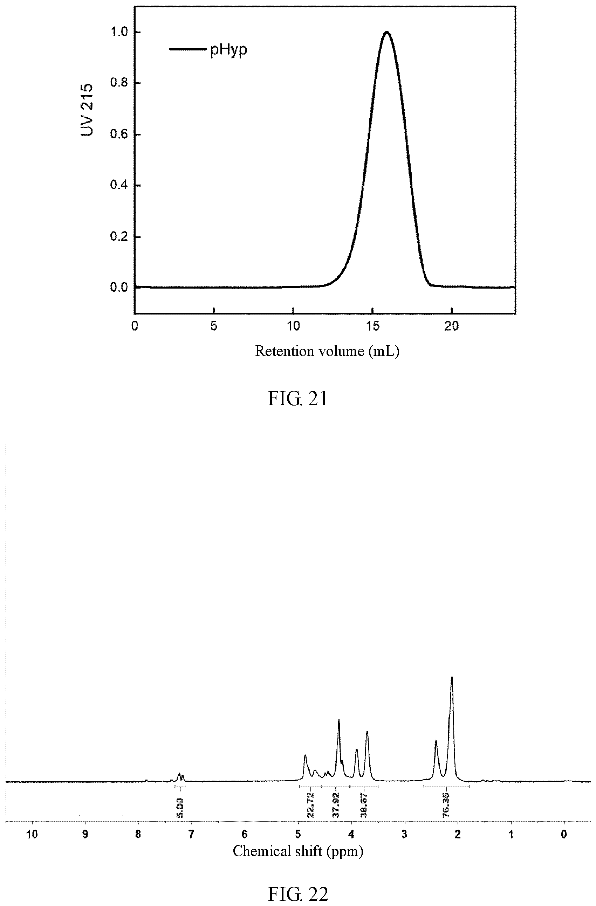

In a 10 °C cold storage, L-hydroxyproline N-carboxyanhydride (150 mg, 0.95 mmol, 20 equivalents) and benzylamine (0.5 M × 95 µL, 0.048 mmol, 1 equivalent) were added into a 5 mL glass bottle, and 750 µL of analytical grade acetonitrile and 750 µL of double-distilled water were added. The mixture was reacted for 10 minutes. The product was passed through a PD 10 desalting column and lyophilized to obtain the final product L-polyhydroxyproline (73 mg, yield 68%). Figure 21 is a size exclusion chromatography (SEC) chart of L-polyhydroxyproline.

The hydrogen and carbon NMR spectra of L-polyhydroxyproline are as follows:
**¹H NMR** (400 MHz, Deuterium Oxide) δ 4.90 - 4.83 (m, 1H), 4.68 - 4.58 (m, 1H), 3.96 - 3.84 (m, 1H), 3.81 - 3.69 (m, 1H), 2.47 - 2.29 (m, 1H), 2.09 - 1.92 (m, 1H).
**¹³C NMR** (101 MHz, D₂O) δ 171.4, 128.8, 127.2, 69.9, 57.3, 54.8, 35.6.

### Example 5: Copolymerization of HypNCA, ProNCA and GlyNCA

In a 10 °C cold storage, L-hydroxyproline N-carboxyanhydride (21 mg, 0.136 mmol, 17 equivalents), L-proline N-carboxyanhydride (19 mg, 0.138 mmol, 17 equivalents), glycine N-carboxyanhydride (14 mg, 0.139 mmol, 17 equivalents) and benzylamine (0.5 M × 16 µL, 0.008 mmol, 1 equivalent) were weighed and placed in a 5 mL glass bottle. 300 µL of analytical grade acetonitrile and 300 µL of double-distilled water were added. The mixture was pipetted quickly to be fully dissolved, and reacted for 10 minutes. The product was passed through a PD 10 desalting column and lyophilized to obtain the final copolymer (21 mg, yield 58%). Figure 22 is the hydrogen nuclear magnetic resonance spectrum of the copolymer product of HypNCA, ProNCA and GlyNCA.

### Example 6: Copolymerization of ProNCA, AlaNCA and SerNCA

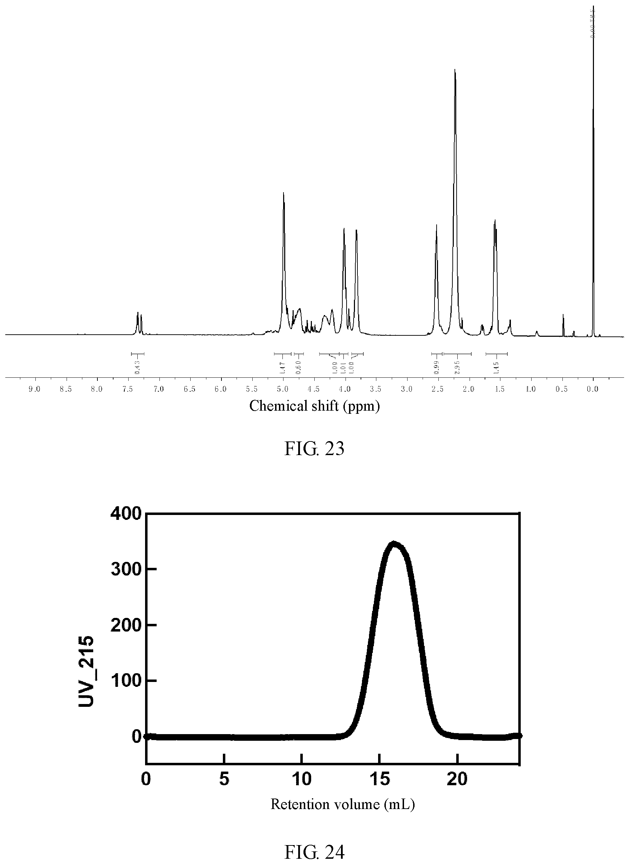

In a 10 °C cold storage, L-proline N-carboxyanhydride (14 mg, 0.100 mmol, 25 equivalents), L-alanine N-carboxyanhydride (12 mg, 0.100 mmol, 25 equivalents), and L-serine N-carboxyanhydride (26 mg, 0.200 mmol, 50 equivalents) were weighed and placed in a 5 mL glass bottle. 250 µL of analytical grade acetonitrile and 250 µL of double-distilled water were added. The mixture was stirred with a magnet bar to be fully dissolved. Then benzylamine (0.5 M × 8 µL, 0.004 mmol, 1 equivalent) was added, and the reaction was carried out for 2 hours. Subsequently, the reaction mixture was dialyzed in water and lyophilized to obtain the final copolymer (18 mg, yield 35%). Figures 23 and 24 show the spectra and aqueous SEC charts of ProNCA, AlaNCAand SerNCA copolymers, respectively.

### Example 7: Preparation of L-polyproline-protein conjugates or L-polyhydroxyproline-protein conjugates.

1. Green fluorescent protein (GFP) was used as an initiator to initiate the polymerization of ProNCA or HypNCA in PBS buffer solution to obtain a GFP-polyamino acid conjugate with adjustable molecular weight.

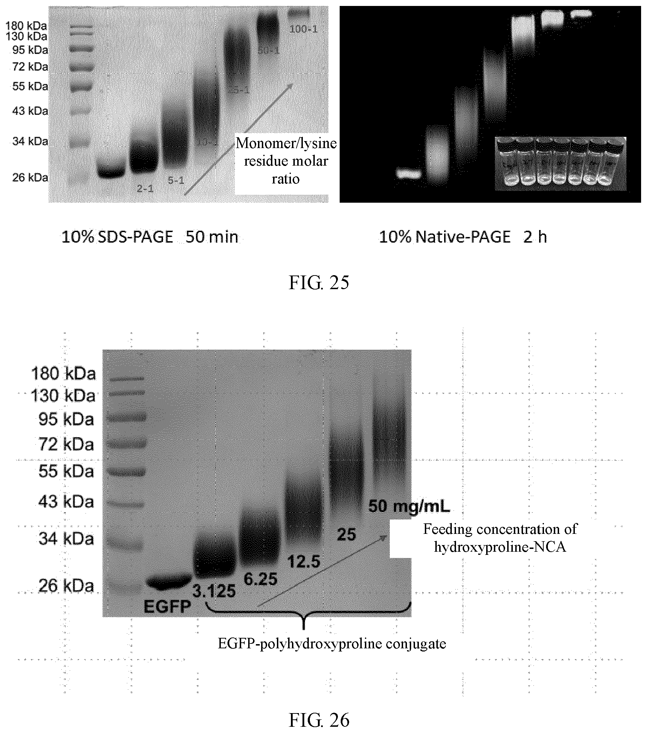

In a 10 °C cold storage, L-proline N-carboxyanhydride was formulated into 50 mg/mL, 25 mg/mL, 12.5 mg/mL, 5 mg/mL, 2.5 mg/mL, and 1 mg/mL solutions in acetonitrile. Then, 100 µL of solutions of L-proline N-carboxyanhydride in acetonitrile with different concentrations were mixed with 100 µL of 5 mg/mL solution of green fluorescent protein in PBS, and reacted for 10 minutes. The product was analyzed using denaturing polyacrylamide gel chromatography (SDS-PAGE) and confirmed that a range of molecular weights of L-polyproline-protein conjugates were obtained. The results are shown in Figure 25. The monomer is the input L-proline N-carboxyanhydride, and the lysine residue is the lysine residue of green fluorescent protein. As the molar ratio between the two increases, the molecular weight of the protein-polyamino acid conjugate obtained increases, indicating that the polymerization of monomers and proteins can be controlled by the ratio of monomers to lysine residues.

2. Preparation of L-polyhydroxyproline-protein conjugate: In a 10 °C cold storage, L-hydroxyproline N-carboxyanhydride was formulated into 50 mg/mL, 25 mg/mL, 12.5 mg/mL, 6.25 mg/mL, 3.125 mg/mL solutions in acetonitrile. Then, 50 µL of solutions of L-hydroxyproline N-carboxyanhydride in acetonitrile with different concentrations were mixed with 100 µL of 5 mg/mL solution of green fluorescent protein in PBS, and reacted for 10 minutes. The product was analyzed using denaturing polyacrylamide gel chromatography (SDS-PAGE) and confirmed that a range of molecular weights of L-polyhydroxyproline-protein conjugates were obtained.

The SDS-PAGE pattern of L-polyhydroxyproline-protein conjugates is shown in Figure 26. The molecular weight of the L-polyhydroxyproline-protein conjugate can be increased by increasing the feeding concentration of L-hydroxyproline N-carboxyanhydride, indicating that the conjugate is polymerized in a controlled manner.

3. Using dihydrofolate reductase (DHFR) as an initiator, ProNCA polymerization was initiated in PBS buffer solution to obtain a DHFR-polyproline conjugate with an adjustable molecular weight, and the enzyme activity was maintained.

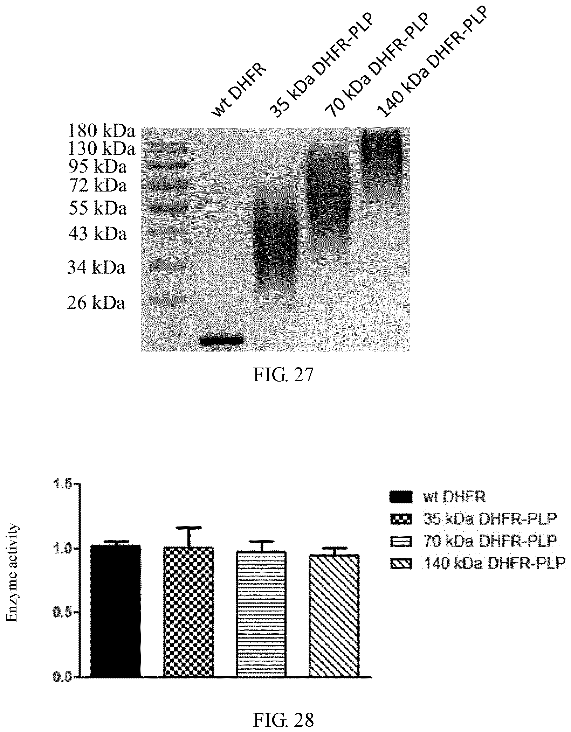

In a 10 °C cold storage, L-proline N-carboxyanhydride was formulated into 5 mg/mL, 10 mg/mL, and 20 mg/mL solutions in acetonitrile. Then, 100 µL of different concentration solutions of L-proline N-carboxyanhydride in acetonitrile was mixed with 100 µL of 4.6 mg/mL solution of dihydrofolate reductase in PBS, and the mixture was reacted for 10 minutes. The product was analyzed using denaturing polyacrylamide gel chromatography (SDS-PAGE) and confirmed that a range of molecular weights of L-polyproline-protein conjugates were obtained, as shown in Figure 27.

Test method for DHFR enzyme activity: 50 mM phosphate buffer (pH 7.5) was added to 5mM mercaptoethanol as an assay buffer solution, and NADPH and FAH2 were dissolved in the assay buffer solution. 140 µL of a substrate solution (containing 0.25 mM NADPH and 0.25 mM FAH2) was mixed with 10 µL of 18 nM wild-type DHFR or DHFR-PLP conjugate, and the mixture was reacted at 25 °C for 15 min. During this period, 340 nm was used to monitor once every 30 s (the decrease in the absorbance value at 340 nm represents the consumption of the substrate). It was found that the conjugate retained the enzyme activity of the wild type, as shown in Figure 28.

### Example 8: Polymerization of selenium-containing NCA (EG3-SeHC NCA and EG4-SeHC NCA) initiated in situ by EGFP

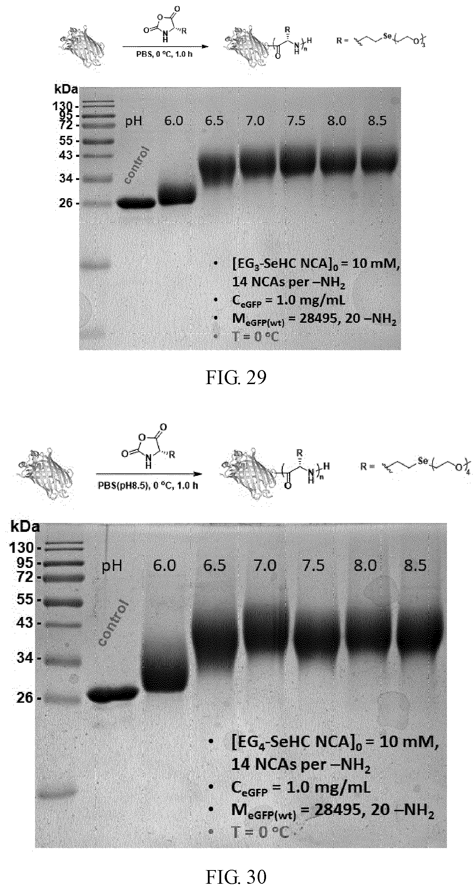

At 0 °C, EG₃-SeHC NCA (0.33 mg, 1 µmol) was weighed. 100 µL of 1 mg/mL EGFP in PBS buffer solution with different pH values was mixed directly with NCA. The product was analyzed using denaturing polyacrylamide gel chromatography (SDS-PAGE) and confirmed that a range of molecular weights of polyselenoamino acid-protein conjugates were obtained. The results are shown in Figure 29. The results indicate that better polymerization effects can be obtained at pH 6-8.5.

At 0 °C, EG₄-SeHC NCA (0.37 mg, 1 µmol) was weighed. 100 µL of 1 mg/mL EGFP in PBS buffer solution (different pH values, 6.0-8.5) was mixed directly with NCA. The product was analyzed using denaturing polyacrylamide gel chromatography (SDS-PAGE) and confirmed that a range of molecular weights of polyselenoamino acid-protein conjugates were obtained. The results are shown in Figure 30. The results indicate that better polymerization effects can be obtained at pH 6-8.5.

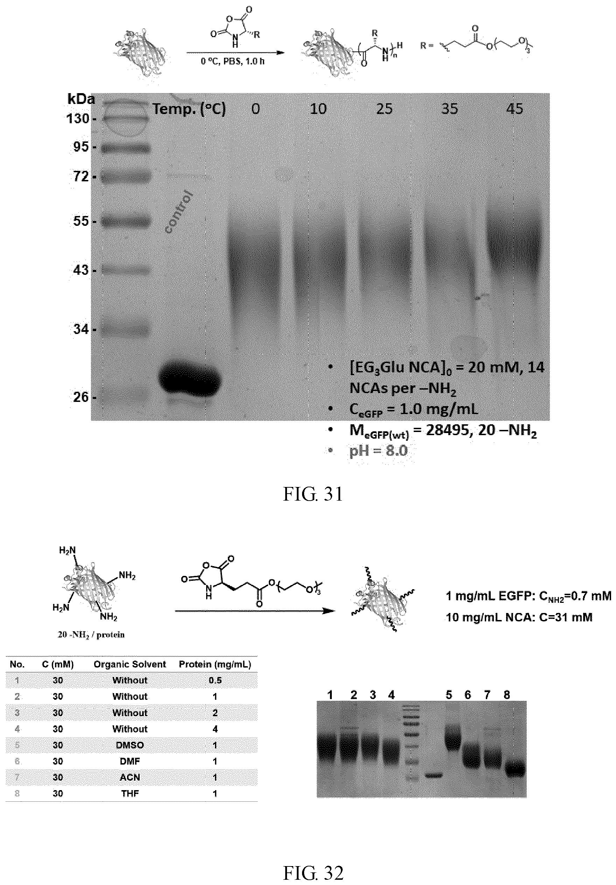

At different temperatures, NCA (0.66 mg, 2 µmol) was weighed. 100 µL of 1 mg/mL EGFP in PBS buffer solution (pH 8.0) was mixed directly with NCA. The product was analyzed using denaturing polyacrylamide gel chromatography (SDS-PAGE) and confirmed that a range of molecular weights of polyselenoamino acid-protein conjugates were obtained. The results are shown in Figure 31. The results indicate that good polymerization effects can be obtained at 0-45 °C.

### Example 9: Polymerization of glutamic acid-derived NCA (EG₃Glu NCA) initiated by EGFP in situ in water or a mixed solvent of water and an organic solvent

At room temperature, 100 µL of EGFP in PBS buffer solution (pH 7.4, Figure 32, No. 1-4, different protein concentrations, 0.5-4 mg/mL) was mixed directly with 1 mg of NCA. The product was analyzed using denaturing polyacrylamide gel chromatography (SDS-PAGE) and confirmed that a range of molecular weights of polyglutamic acid derivative-protein conjugates were obtained.

At room temperature, 100 µL of 1 mg/mL EGFP in PBS buffer solution (pH 7.4) was mixed with 100 µL of 10 mg/mL NCA solution (Figure 32, No. 5-8, different solvents, DMSO, DMF, ACN, THF, 50% water content). The product was analyzed using denaturing polyacrylamide gel chromatography (SDS-PAGE) and confirmed that a range of molecular weights of polyglutamic acid derivative-protein conjugates were obtained. The results, shown in Figure 32, demonstrate that various mixtures of DMSO, DMF, ACN or THF and water can be used in the methods of the present disclosure, and the maximum polymer molecular weight was obtained in the DMSO lane.

### Example 10: Biological functions

### Extended circulation time: Asparaginase-polyproline conjugate has a longer circulation time than wild-type asparaginase

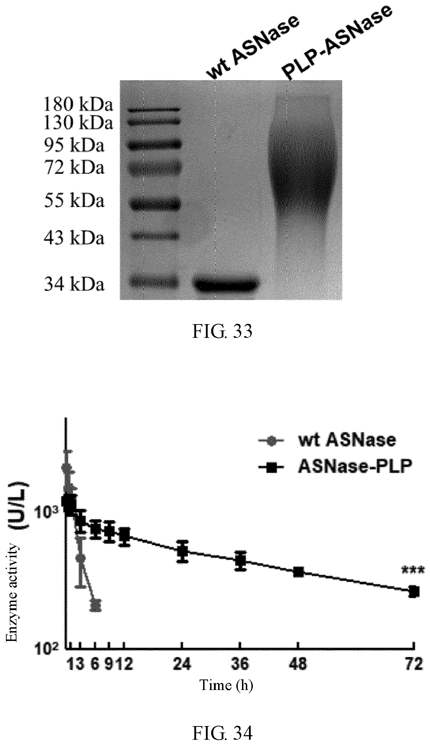

In a 10 °C cold storage, 500 µL of 12 mg/mL solution of L-proline N-carboxyanhydride in acetonitrile was mixed with 500 µL of 4.75 mg/mL solution of asparaginase in PBS. The mixture was reacted for 10 minutes, and purified by a size exclusion chromatography column Superdex 200 increase 10/300 GL. The product was analyzed using denaturing polyacrylamide gel chromatography (SDS-PAGE), as shown in Figure 33, confirming that the asparaginase-polyproline conjugate was obtained.

Assay method: SD rats were randomly divided into 2 groups (n = 3), and 80 µg of wild-type asparaginase or asparaginase-polyproline conjugate was injected into the tail vein. Blood was collected from the orbit at each time point, and the obtained blood was placed on ice to coagulate, and then centrifuged at 4500 rpm for 15 min to obtain serum. In a transparent 96-well plate, 90 µL of phosphate buffer solution, 10 µL of serum to be tested, and 20 µL of substrate L-asparagine solution were added to each well, and reacted at 37°C for 30 min. The reaction was then stopped with 20 µL of 1.5 M trichloroacetic acid. In another transparent 96-well plate, 160 µL of phosphate buffer, 20 µL of the above reaction solution, and then 20 µL of Nessler's reagent were added to each well, and mixed well. The absorbance value was detected at 410 nm.

In the pharmacokinetic assay of tail vein injection in SD rats, wild-type asparaginase was metabolized very quickly with a half-life of only 1.4 hours, while the half-life of the asparaginase-polyproline conjugate was as long as 26 hours, and the enzyme activity can still be detected in the serum until 72 hours. The results are shown in Figure 34.

### Reduced immunogenicity: Asparaginase-polyproline conjugate has lower immunogenicity than that of wild-type asparaginase

Immunization method: SD rats were randomly divided into 3 groups, with 4 rats in each group, two males and two females. Each group was injected subcutaneously with wild-type asparaginase or asparaginase-polyproline conjugate, with an immunization dose of 0.8 mg ASNase/kg, once a week, and a total of four immunizations. Before each immunization, and one week after the last immunization, blood was collected from the orbit. About 200 µL of serum was collected, aliquoted, and frozen at -80 °C for storage.

The antibody content in the serum after immunization was analyzed by enzyme-linked immunoassay (ELISA): wild-type asparaginase was diluted to 1.0 µg/mL with 1 × PBS, added to a 96-well plate (100 µL/well, that is, 100 ng/well), and placed at 4 °C overnight to allow protein adsorption. Each well was washed three times with 200 µL of washing buffer solution, and 100 µL of assay buffer solution was added. The plate was blocked at room temperature for more than 2 hours. The serum collected from the above immunization (0 to 4 weeks) was diluted 5000 times with assay buffer solution, and 100 µL was then added to each well. The plate was shaken on a microplate shaker at room temperature for 1 h, and then washed three times with washing buffer. A secondary antibody (Goat anti-rat IgG HRP) labeled with horseradish peroxidase diluted 5000 times with assay buffer solution was then added, 100 µL per well, and incubated with shaking at room temperature for 1 h. After the incubation, the washing step was repeated, and TMB chromogenic solution (100 µL/well) was added for color development for 5 minutes. 2 N H2SO4 (100 µL/well) was added to terminate the color development, and the absorption value at 450 nm was read on a microplate reader.

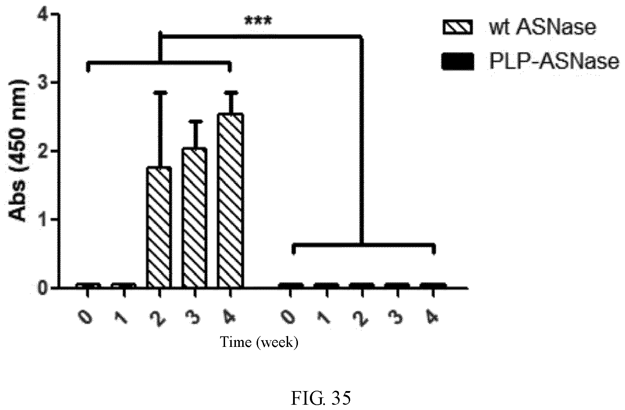

In the in vivo immunogenicity assay in SD rats, polyproline showed a good antigen-shielding effect and greatly reduced the production of antibodies against asparaginase. The results are shown in Figure 35.

### Antifreeze effect: Asparaginase-polyproline conjugate has better antifreeze effect than that of commercially available Pegaspargase.

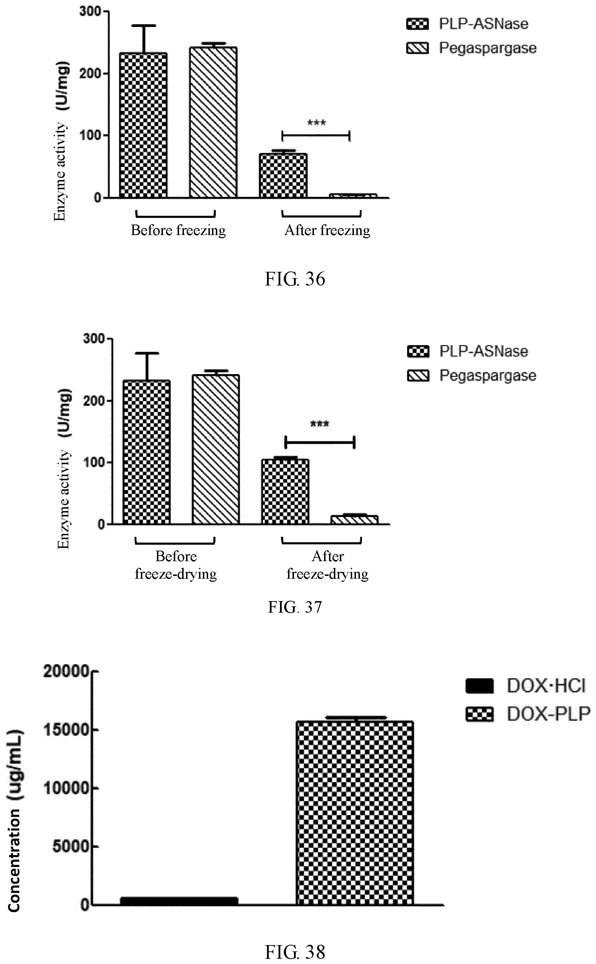

As shown in Figure 36, the cryopreservation method of slow freezing at -20°C reduced pegaspargase enzyme activity to only 3% of its original value, and the asparaginase-polyproline conjugate showed significant differences. Under the same conditions, the enzyme activity of asparaginase-polyproline conjugate was retained by 35%. As shown in Figure 37, the above conjugate was made into lyophilized powder. After adding double-distilled water to reconstitute, the enzyme activity of Pegaspargase was only 6% of the original, and the polyproline conjugate showed significant differences. Under the same conditions, the enzyme activity retention of asparaginase-polyproline conjugate reached 50%.

### Anti-tumor effect

### Asparaginase-polyproline conjugate (ASNase-PLP) retains the in vitro tumor cell growth inhibitory activity of wild-type asparaginase to a large extent

24 hours before treatment, NKYS cells (cultured in-house) were seeded at a density of 5,000 cells per well, and incubated with different concentration gradients of wild-type asparaginase or asparaginase-polyproline conjugate (ASNase-PLP) in a black 96-well plate for 48 hours (n = 3). The relative cell viability of each group was detected using CellTiter-Blue^{®} (Promega, USA). Data fitting and IC₅₀ calculation were analyzed using GraphPad Prism 5.0 software.

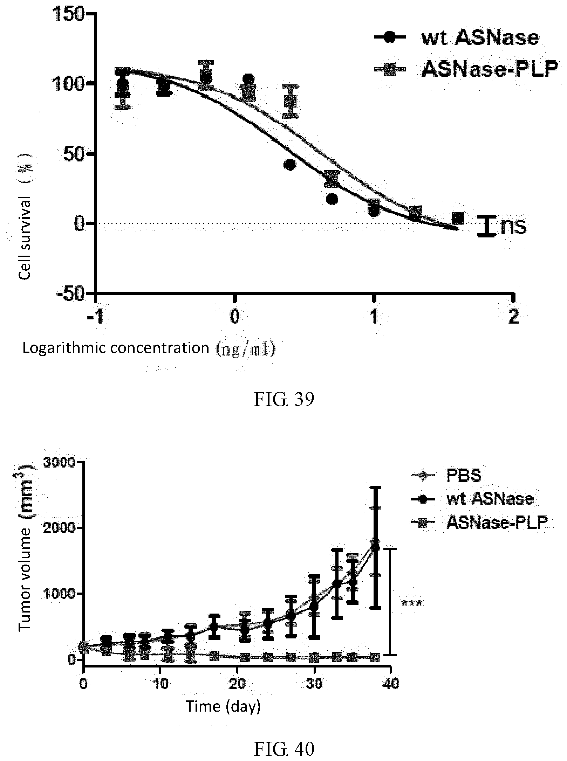

Figure 39 shows a plot of cell viability as a function of concentration. According to Figure 39, the asparaginase-polyproline conjugate (ASNase-PLP) retains the in vitro tumor cell growth inhibitory activity of wild-type asparaginase to a large extent. The IC50 of wild-type asparaginase is 2.4 ng/mL; while the IC50 of ASNase-PLP is 4.3 ng/mL.

### Asparaginase-polyproline conjugate (ASNase-PLP) has significantly improved in vivo anti-tumor activity compared with wild-type asparaginase

NKYS cells (6.0 × 10⁶) suspended in 0.1 mL of PBS were mixed with an equal volume of Matrigel^{™} matrix (Corning, USA) and inoculated subcutaneously into 6-week-old B-NDG mice. When tumors grew to approximately 300 mm³, mice were randomly divided into three groups (n = 8) and treated every two weeks with PBS, wild-type ASNase, or ASNase-PLP via intraperitoneal injection at a dose of 15 U/mouse. The tumor volume was obtained by the formula *V* = *L* × *W²*/2*,* where V, L, and W were the volume, length, and width of the tumor, respectively. Tumor volume and mouse body weight were measured every 3 days.

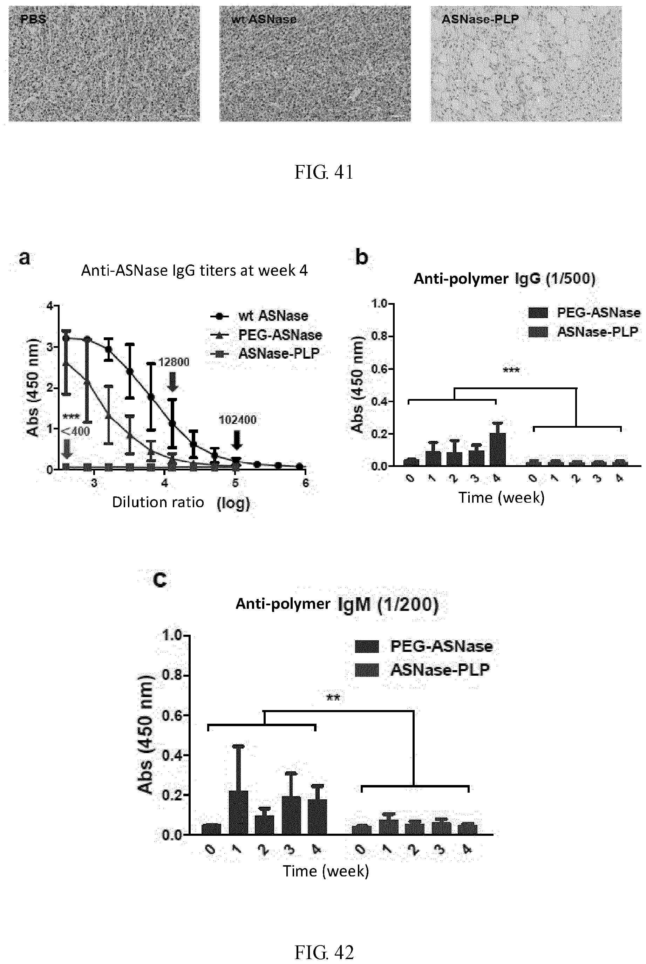

Histopathological evaluation: The mice were sacrificed on day 38. The extracted tumor sections were stained by immunohistochemistry using anti-human Ki-67 antibody to detect rapidly proliferated tumor cells, and imaged on Axio Scan Z1 (Carl Zeiss, Germany). Figure 40 shows a plot of tumor volume as a function of treatment days, showing that asparaginase-polyproline conjugate (ASNase-PLP) has significantly improved in vivo anti-tumor activity compared to wild-type asparaginase. Figure 41 shows photographs of histopathological examination of tumors.

### Immunization and ELISA

Male and female SD rats were randomly divided into groups (n = 4) and received wild-type ASNase, ASNase-PLP, or PEG-ASNase subcutaneously at a dose of 200 U ASNase/kg per week (the preparation method is the same as that in Biomaterials 2021, 268: 120606, which is incorporated by reference in its entirety; obtained by using Traut's reagent to increase the number of active sulfhydryl groups on the surface of asparaginase and coupling to polyethylene glycol with a maleimide end group). Blood was drawn before immunization (day 0) and before each injection (days 7, 14, 28, and 35). The antisera were then assessed for anti-ASNase and anti-polymer antibodies by ELISA.

Antigens used in direct ELISA: wild-type ASNase (for detection of anti-ASNase IgG), PLP-interferon (IFN) conjugate (for detection of anti-PLP antibodies) and PEG-IFN conjugate (the preparation method is the same as that in Bioconjugate Chem. 2018, 29: 2232-2238; obtained by using molecular cloning technology to introduce active cysteine at the N-terminus of interferon, and coupling with polyethylene glycol with a thioester end group) (for detection of anti-PEG antibodies).

ELISA procedure for anti-polymer IgM measurement: polymer (PLP or PEG)-IFN conjugate in PBS (100 µL × 1.0 µg/mL/well) was added to a high-binding clear 96-well plate (Corning, USA) and incubated at 4 °C overnight for antigen coating. The plate was washed with washing buffer solution (a 0.5‰ solution of CHAPS in PBS, 200 µL/well × 3) and blocked with assay buffer solution (5% BSA in washing buffer solution, 100 µL) at room temperature for 2 h. Next, pre-diluted antiserum (100 µL, diluted 200-fold with assay buffer solution) was added to a washed plate and incubated at room temperature for 1 h. Subsequently, the plate was washed three times with washing buffer solution and incubated with goat anti-rat IgM mu chain secondary antibody HRP (source abcam, ab98373) (100 µL, diluted 5000 times with assay buffer solution) at room temperature for 1 h. Finally, after washing four times, the plate was incubated with TMB solution (100 µL, CWBio) at room temperature for color development reaction. After 5 minutes, the reaction was stopped with 2 N H₂SO₄ (100 µL) and the absorbance at 450 nm was read in a plate reader.

ELISA procedure for anti-ASNase IgG and anti-polymer IgG: The procedure was performed similarly to above, but using different coating antigens, dilution times, and buffer formulations. More detailed information is provided in Table 2 below.

**Table 2: ELISA conditions and buffer formulations**

| | Coating antigen | Washing buffer solution | Assay buffer solution | Antiserum dilution time | Secondary Antibody |
|---|---|---|---|---|---|
| Anti-ASNase IgG | 100 ng/well wt ASNase in PBS | 0.5‰ Tween-20 in PBS | 5% BSA and 0.5‰ Tween-20 in PBS | 5000 | Goat anti-rat IgG Fc HRP (ab97090) |
| Antipolymer IgG | 100 ng/well polymer-IFN in PBS | 0.5‰ CHAPS in PBS | 5% BSA and 0.5‰ CHAPS in PBS | 500 | Goat anti-rat IgG Fc HRP (ab97090) |
| Anti-polymer IgM | | | | 200 | Goat anti-rat IgM mu chain HRP (ab98373) |

ELISA procedure for anti-ASNase IgG titer: The procedure was performed similarly to the above, using the 4th week antiserum for gradient dilution (as shown in panel a of Figure 42, the dilution times were 400, 800, 1600, 3200, 6400, 12800, 25600, 102400).

Figure 42 shows (a) a graph of the correlation between different dilution ratios and anti-ASNase IgG at week 4; (b) a graph of changes in anti-polymer IgG at different times; and (c) a graph of changes in anti-polymer IgM at different times. The results show that the asparaginase-polyproline conjugate (ASNase-PLP) has significantly lower immunogenicity than those of wild-type asparaginase (wt ASNase) and asparaginase-polyethylene glycol conjugate. This is reflected in the fact that after multiple immunizations, the serum of mice injected with ASNase-PLP had the lowest anti-asparaginase antibody IgG level (a) and produced less anti-polymer antibody (b-c).

### Example 11: Solubilizing effect: Doxorubicin-polyproline conjugate (DOX-PLP) improves the solubility of doxorubicin

Doxorubicin was used as an initiator to initiate the polymerization of polyproline to obtain DOX-PLP, which can improve the water solubility of DOX. 500 uL of aqueous solution of doxorubicin hydrochloride (9.63 mg, 0.0177 mmol, 1 equivalent) was mixed with a solution of ProNCA in acetonitrile (50 mg, 0.354 mmol, 20 equivalents), and the mixture was stirred at 10°C for 10 minutes to obtain DOX-PLP. Similarly, gemcitabine was used as an initiator to initiate the polymerization of polyproline to obtain a gemcitabine-PLP conjugate.

Determination of doxorubicin content: 600, 300, 150, 75, 37.5, 18.75, 9.375, and 0 ug/mL DOX was formulated as a standard curve. 5 mg of doxorubicin hydrochloride was weighed and added to 1 mL of PBS. 10 mg of DOX-PLP was weighed and added to 100 µL of PBS. After dissolving by vortexing, the mixture was centrifuged at 21,000 for 10 min, and the supernatant was diluted to an appropriate multiple. The absorbance value was measured at 495 nm, and the DOX content was calculated based on the absorbance value of the standard curve.

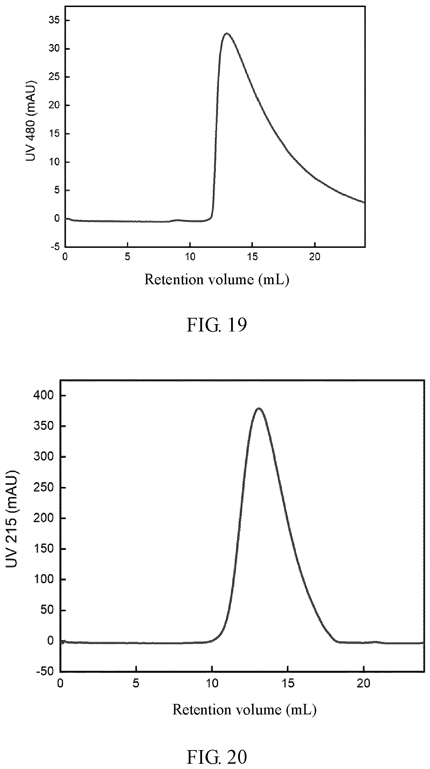

Figure 19 is an SEC chart of doxorubicin-PLP conjugate. As shown in Figure 38, the solubility limit of doxorubicin hydrochloride in PBS is 573±22 ug/mL DOX, and DOX-PLP can reach 15716±697 ug/mL DOX, which is a 27-fold increase.

### Example 12: Uricase-polyproline conjugate (UOx-PLP) retains wild-type uricase activity, improves thermal stability in vitro and circulation time in vivo, and reduces immunogenicity in vivo.

### Preparation of uricase-polyproline conjugate (UOx-PLP)

### Preparation and basic characterization

In a 10 °C cold storage, 500 µL of 7.4 mg/mL solution of L-proline N-carboxyanhydride in acetonitrile was mixed with 500 µL of 2.0 mg/mL solution of uricase in PBS (pH 7.4), and the mixture was reacted for 5 minutes. The product was purified by a size exclusion chromatography column Superdex 200 increase 10/300 GL. The product was analyzed using denaturing polyacrylamide gel chromatography (SDS-PAGE).

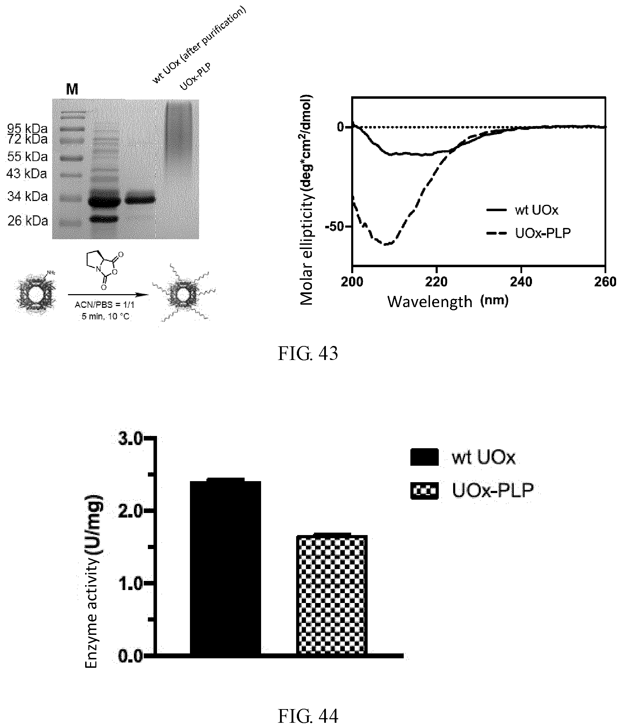

As shown in the left panel of Figure 43, it was confirmed that the uricase-polyproline conjugate (UOx-PLP) was obtained.

Circular dichroism spectroscopy test method: A 0.2 mg/mL solution (pH 7.4) of wild-type uricase and uricase-polyproline conjugate in PBS was formulated, and the scanning range was 200-260 nm (instrument name JASCO J-815). As shown in the right panel of Figure 43, the conjugate exhibits characteristics of a water-soluble polyproline helix (PPII).

### Enzyme activity

### Principle and method of uricase enzyme activity test:

Uricase catalyzes the decomposition of uric acid into allantoin, carbon dioxide, and hydrogen peroxide. Hydrogen peroxide can oxidize Fe2+ in potassium ferrocyanide to generate Fe3+. Fe3+ further combines with 4-aminoantipyrine and phenol to generate a red quinone compound with a characteristic absorption peak at 505 nm.

30 µL of wild-type uricase or uricase-polyproline conjugate solution and hydrogen peroxide standard solution were mixed well with 170 µL of test working solution, and the mixture was incubated at 25°C for 30 minutes. The absorbance value at 505 nm was determined (n=3). Then, the uricase activity was converted according to the standard solution. The enzyme required to decompose 1 µmol of uric acid per minute at 25°C and pH 8.8 is defined as one unit (1 U).

As shown in Figure 44, the conjugate retained the wild-type enzyme activity.

### In vitro thermal stability

### Determination of unfolding temperature:

This assay used SYPRO^{®}Orange, a fluorescent dye that can interact with the hydrophobic domain of proteins, combined with programmed temperature rise, to measure the unfolding temperature of the sample.

15 µL of protein dye was mixed with 135 µL of wild-type uricase or polyproline conjugate solution to obtain an assay sample with a final concentration of 5 µM and containing 20×dye. The assay sample was added into a white eight-row tube (50 µL per well, n=3). The white eight-row tube was placed in a fluorescence quantitative PCR instrument, and the program was set to increase the temperature from 37°C to 98°C at a constant rate, with an increase of 2.2°C per minute.

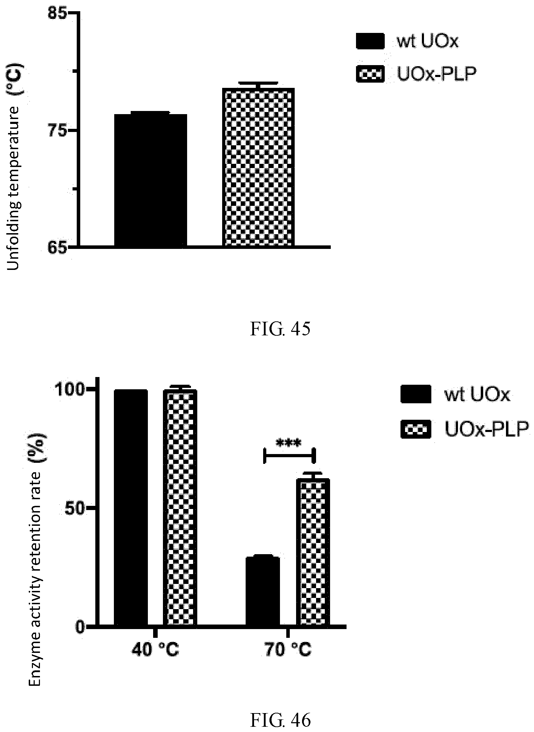

As shown in Figure 45, the unfolding temperature of uricase-polyproline conjugate is higher than that of wild-type uricase.

Determination of enzyme activity retention after heating:
Wild-type uricase or polyproline conjugate solution was added in an eight-row tube. A PCR instrument was used for programmed temperature control. After incubation at 40°C or 70°C for 30 minutes, the temperature was immediately lowered to 4°C. Subsequently, enzyme activity detection was performed as described above (n=3).

As shown in Figure 46, under thermal stimulation, the enzyme activity retention of uricase-polyproline conjugate was significantly improved compared with wild-type uricase.

### Internal circulation time

SD rats were randomly divided into 2 groups (n=3), and wild-type uricase or uricase-polyproline conjugate was injected through the tail vein at a dose of 0.6 mg UOx/kg. Blood was collected from the orbit at each time point. The obtained blood was placed on ice to coagulate, and then centrifuged at 4500 rpm for 15 min to obtain serum. In a transparent 96-well plate, 30 µL of serum or hydrogen peroxide standard solution was mixed well with 170 µL of a working solution for the enzyme activity test, and the mixture was incubated at 25 °C for 30 minutes. The absorbance value at 505 nm was measured, and the half-life was determined by the retention of enzyme activity.

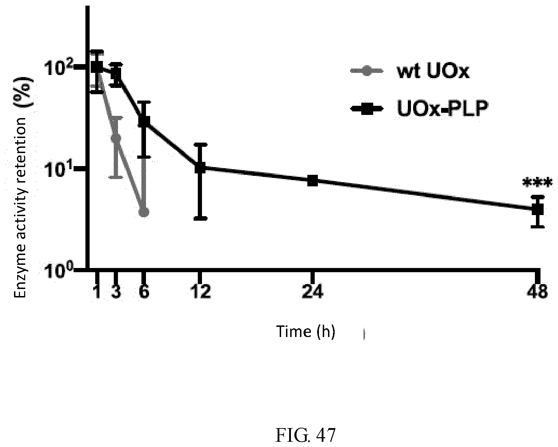

In the pharmacokinetic assay of tail vein injection in SD rats, wild-type uricase was metabolized very quickly with a half-life of only 2.2 hours, while the half-life of uricase-polyproline conjugate was as long as 11 hours. The results are shown in Figure 47.

### Immunogenicity

Immunization method: SD rats were randomly divided into 3 groups, with 4 rats in each group, 2 females and 2 males. Each group was injected subcutaneously with wild-type uricase wt Uox, polyethylene glycol-uricase conjugate PEG-UOx (the preparation method was the same as Biomaterials 2021, 268: 120606. Obtained by using Traut's reagent to increase the number of active thiol groups on the surface of uricase, and coupling with polyethylene glycol with a maleimide end group), and uricase-polyproline conjugate UOx-PLP. The immunization dose was 0.6 mg UOx/kg, the frequency was once a week, and the immunization was four times in total. Before each immunization and one week after the last immunization, blood was taken from the orbit. Serum was collected and frozen at -80°C.

The antibody content in the serum after immunization was analyzed by enzyme-linked immunoassay (ELISA): wild-type uricase was diluted to 1.0 µg/mL with 1 × PBS, added to a 96-well plate (100 µL/well, that is, 100 ng/well), and placed at 4 °C overnight. Each well was washed three times with 200 µL of washing buffer, 100 µL of assay buffer solution was added, and the plate was blocked at room temperature for more than 2 h. The above immunized serum (0 to 4 weeks) was diluted 10000 times (anti-UOx IgG assay) or 500 times (anti-UOx IgM assay) with assay buffer solution, and then 100 µL was added to each well. After shaking at room temperature on a microplate shaker for 1 hour, the plate was washed three times with washing buffer solution, and then a secondary antibody (Goat anti-rat IgG HRP or Goat anti-rat IgM HRP) labeled with horseradish peroxidase diluted 10,000 times with assay buffer solution was added, 100 µL per well. The plate was incubated with shaking at room temperature for 1 h. After the incubation, the washing step was repeated. TMB chromogenic solution (100 µL/well) was added for color development for about 3 minutes. 2 N H2SO4 (100 µL/well) was added to stop color development, and the absorbance at 450 nm was read on a microplate reader.

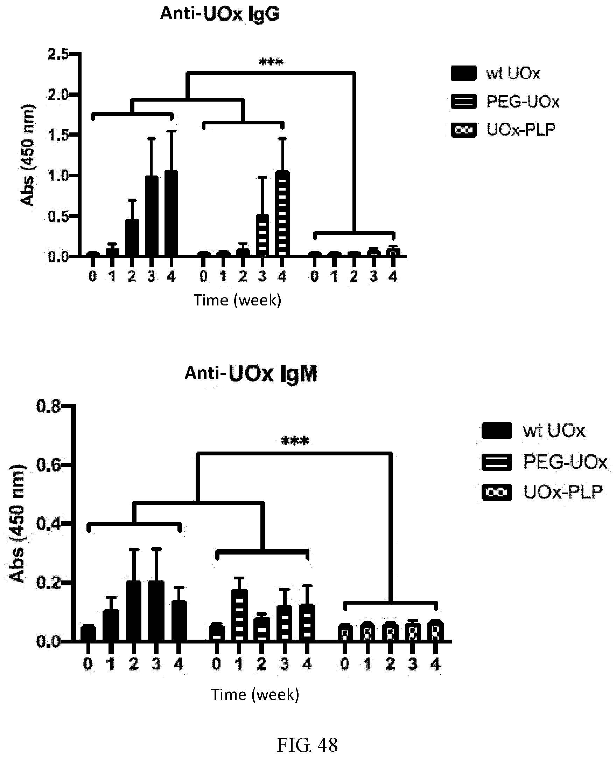

In the immunogenicity assay in SD rats, polyproline showed a good antigen-shielding effect and greatly reduced the production of antibodies against uricase. The results are shown in Figure 48.

### Long-term in vivo safety evaluation

Polyproline synthesis (PLP₂₅, PLP₅₀, PLP₁₀₀): PLP₁₀₀ was used as an example.

In a 10 °C cold storage, L-proline N-carboxyanhydride (200 mg, 1.4 mmol, 100 equiv.) and L-proline (1 M × 14 µL, 0.014 mmol, 1 equiv.) were added to a 20 mL glass bottle. 1 mL of analytical grade acetonitrile and 1 mL of 1×PBS were added, and the mixture was reacted for 10 minutes. The product was passed through a PD 10 desalting column, and freeze dried to obtain the final product. The initiator ratio was adjusted to obtain polyproline with different molecular weights, PLP₂₅, PLP₅₀, and PLP₁₀₀.

SD rats were randomly divided into 4 groups (n=3), and PBS solution and three proline polymers with different molecular weights (PLP₂₅, PLP₅₀, PLP₁₀₀) were respectively administered via tail vein administration. The dosage was 30 mg PLP/kg (the same volume was administered to the PBS control group), and the frequency was once a week, for a total of twelve times. Weight data were recorded regularly.

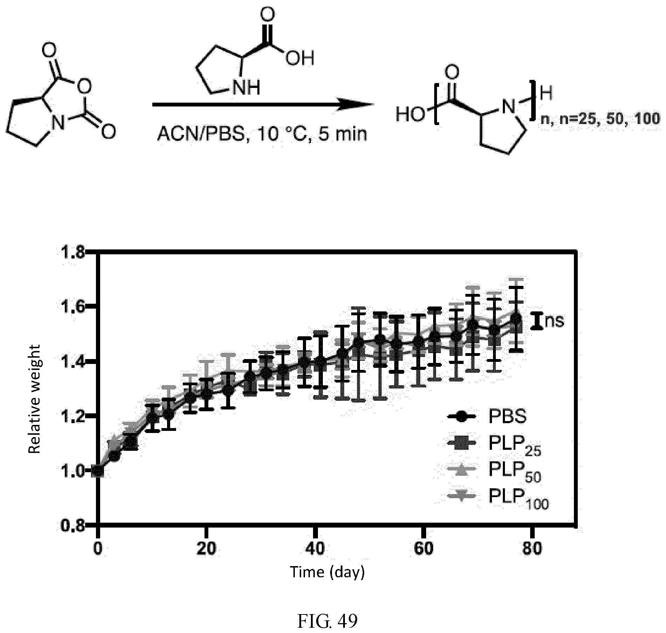

During the three-month long-term weight monitoring, there was no significant difference between each polyproline administration group and the PBS control group, indicating that polyproline has good biological safety, as shown in Figure 49.

It should be understood that, while the present disclosure has been described in connection with its detailed description, the foregoing description is intended to illustrate rather than limit the scope of the disclosure, which is defined by the scope of the appended claims. Other aspects, advantages and modifications are within the scope of the appended claims.

## Claims

1. A method for polymerizing amino acid N-carboxyanhydrides, which includes adding one or more amino acid N-carboxyanhydrides in water or a mixed solution of water and an organic solvent in the presence of an initiator to react to produce an initiator-polyamino acid conjugate, and the organic solvent is selected from the group consisting of: acetonitrile, pyridine, N,N-dimethylformamide, tetrahydrofuran and dimethyl sulfoxide; preferably, the degree of polymerization and molecular weight of the initiator-polyamino acid conjugate are controlled by adjusting the ratio of the initiator and the amino acid N-carboxyanhydride monomer.

2. The method of claim 1, wherein the initiator is a small molecule or large molecule containing a nucleophilic group selected from the group consisting of amino, imino, guanidyl, hydroxyl and sulfhydryl;
preferably, wherein the large molecule is a protein, polypeptide or nucleic acid, preferably the protein is selected from the group consisting of enzymes, antibodies, cytokines and marker proteins, such as fluorescent proteins, asparaginase, esterase, azoreductase, uricase, dihydrofolate reductase, phenylalanine ammonia lyase, cystathionine beta synthase or hyaluronidase;
preferably, wherein the small molecule is a small molecule drug, probe or dye, preferably, wherein the small molecule drug is doxorubicin or gemcitabine.

3. The method of any one of claims 1-2, wherein the water or the mixed solution of water and an organic solvent has a salt, such as a buffer salt, preferably one or more selected from the group consisting of sodium salt, potassium salt, magnesium salt and ammonium salt.

4. The method of any one of claims 1-3, wherein the amino acid is selected from natural amino acids and unnatural amino acids,
preferably wherein said amino acid comprises glycine, alanine, valine, leucine, isoleucine, methionine, proline, tryptophan, serine, tyrosine, cysteine, phenylalanine, asparagine,
glutamine, threonine, aspartic acid, sarcosine, glutamic acid, lysine, arginine, histidine, ε-nitrogen-benzyloxycarbonyl lysine, glutamic acid benzyl ester, hydroxyproline, (S)-2-amino-4-((3-(benzylamino)-3-oxypropyl)seleno)butyric acid, penicillamine, oxy-tert-butyl-serine, ε-nitrogen-trifluoroacetyl L-lysine, or homocysteine or derivatives of these amino acids, for example, derivatives in which the sulfhydryl group in homocysteine is replaced by a selenoalkoxy group, preferably wherein the amino acid is selected from methionine, valine, leucine, isoleucine, proline, hydroxyproline and alanine or derivatives of these amino acids; preferably wherein the polyamino acid is a homopolymer or copolymer of the amino acids, preferably a random copolymer.

5. The method of any one of claims 1-4, wherein the small molecule is selected from the group consisting of benzylamine, ethylenediamine, p-methylaniline, lysine, glucosamine, p-methylthiophenol, mercaptan, doxorubicin, and gemcitabine.

6. The method of any one of claims 1 to 5, wherein the amino acid N-carboxyanhydride is selected from the group consisting of hydroxyproline N-carboxyanhydride, proline N-carboxyanhydride, glycine N-carboxyanhydride, alanine N-carboxyanhydride and serine N-carboxyanhydride.

7. A method for producing protein-poly amino acid conjugates, which includes adding one or more amino acid N-carboxyanhydrides in water or a mixed solution of water and an organic solvent in the presence of a protein to react to produce a protein-polyamino acid conjugate, the organic solvent is selected from the group consisting of: acetonitrile, pyridine, N,N-dimethylformamide, tetrahydrofuran and dimethyl sulfoxide;
preferably, wherein the water content is 20-100 v/v%;
preferably, wherein the reaction is carried out at -20 to 65 °C;
preferably, wherein the water or the mixed solution of water and an organic solvent has a salt, such as buffer salts, preferably one or more selected from sodium salts, potassium salts, magnesium salts and ammonium salts;
preferably, wherein the amino acid is selected from natural amino acids or unnatural amino acids, preferably the amino acid includes glycine, alanine, valine, leucine, isoleucine, methionine, proline, tryptophan, serine, tyrosine, cysteine, phenylalanine, asparagine, glutamine, threonine, aspartic acid, sarcosine, glutamic acid, lysine, arginine, histidine, ε-nitrogen-benzyloxycarbonyl lysine, glutamic acid benzyl ester, hydroxyproline, (S)-2-amino-4-((3-(benzylamino)-3-oxypropyl)seleno)butyric acid, penicillamine, oxy-tert-butyl-serine, ε-nitrogen-trifluoroacetyl L-lysine, or homocysteine or derivatives of these amino acids, for example, derivatives in which the sulfhydryl group in homocysteine is replaced by a selenoalkoxy group, preferably wherein the amino acid is selected from proline or its derivatives, homocysteine or its derivatives, glutamic acid or its derivatives, such as derivatives in which the mercapto group in homocysteine is replaced by selenoalkoxyalkyl; preferably, the selenoalkoxyalkyl is -Se-(CH2CH2O)n-alkyl; derivatives in which the hydrogen in the hydroxyl group of glutamic acid side chain is replaced by an alkoxyalkyl group, and the alkoxyalkyl group is (CH2CH2O)n-alkyl; wherein n is preferably an integer of 2 or more, such as 3 or 4;
preferably, wherein the proline derivative is: 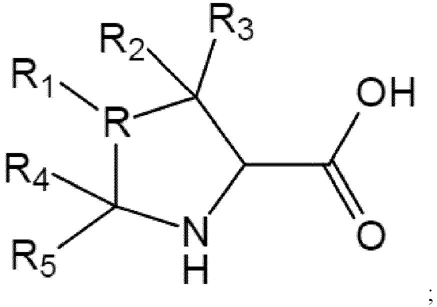
wherein R is CH, N, O, S or Se; R₁ is absent, hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl;
R₂, R₃, R₄ and R₅ are each independently hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, optionally substituted C₆-C₁₂ aryl, or optionally substituted C₆-C₁₂ heteroaryl;
preferably, wherein the proline derivative is: 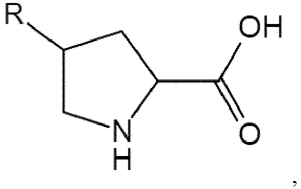
wherein R is hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy group or optionally substituted C₆-C₁₂ aryl group or optionally substituted C₆-C₁₂ heteroaryl group;
or 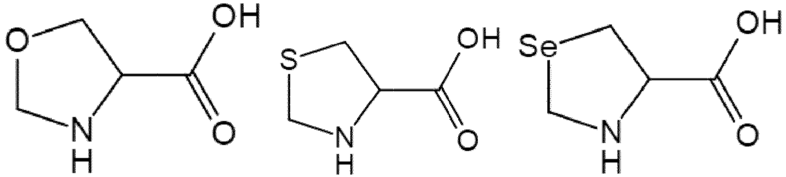 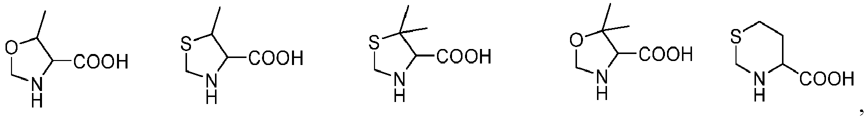 preferably, the protein is selected from the group consisting of enzymes, antibodies, cytokines and marker proteins, such as green fluorescent protein, asparaginase, esterase, azoreductase, uricase or dihydrofolate reductase; preferably wherein the polyamino acid is a homopolymer or copolymer of the amino acids, preferably a random copolymer.

8. Use of a polyamino acid in prolonging the circulation time of proteins or polypeptides in the body, reducing the immunogenicity of proteins or polypeptides, serving as antifreeze for proteins or polypeptides, or increasing the solubility of drugs, wherein the polyamino acid is coupled to a protein or drug by the method of any one of claims 1-7, preferably wherein the polyamino acid is selected from: polyproline or a proline derivative or a copolymer of ProNCA, AlaNCA and SerNCA; preferably wherein the protein is selected from enzymes, antibodies, cytokines and marker proteins, such as fluorescent proteins, asparaginase, esterase, azoreductase, uricase, dihydrofolate reductase, phenylalanine ammonia lyase, cystathionine beta synthase and hyaluronidase; preferably, wherein the small molecule is a small molecule drug, probe or dye, preferably, wherein the small molecule drug is doxorubicin or gemcitabine; preferably, wherein the proline derivative is: 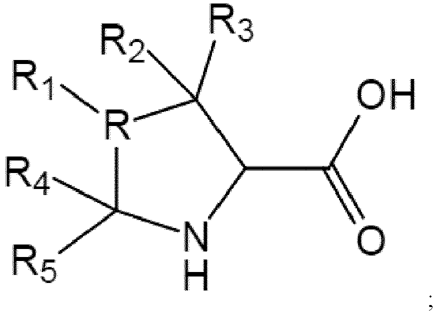
wherein R is CH, N, O, S or Se; R₁ is absent, hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl;
R₂, R₃, R₄ and R₅ are each independently hydrogen, hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, optionally substituted C₆-C₁₂ aryl, or optionally substituted C₆-C₁₂ heteroaryl;
preferably, the proline derivative is: 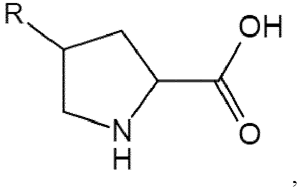
wherein R is hydroxyl, halogen, optionally substituted C₁₋₆ alkyl, optionally substituted C₂₋₆ alkenyl, optionally substituted C₂₋₆ alkynyl, optionally substituted C₁₋₆ heteroalkyl, C₂₋₆ heteroalkenyl, C₂₋₆ heteroalkynyl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₆R₇, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₆ and R₇ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl; or

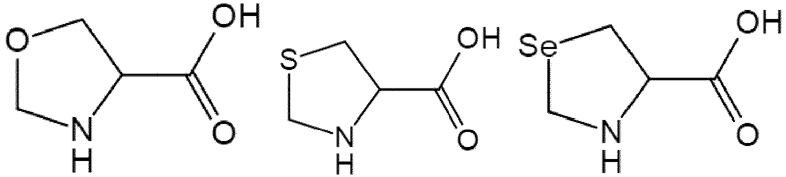

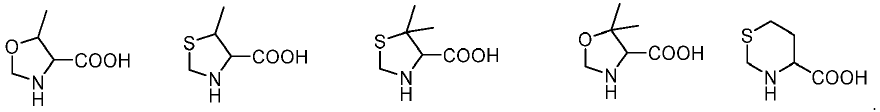

9. A conjugate produced by the method of any one of claims 1-6, wherein the initiator is a small molecule or large molecule containing a nucleophilic group selected from amino, imino, guanidyl, hydroxyl, and sulfhydryl; preferably, wherein the large molecule is a protein, a polypeptide or a nucleic acid, preferably, wherein the protein is selected from enzymes, antibodies, cytokines and marker proteins, such as fluorescent proteins, asparaginase, esterase, azoreductase, uricase, dihydrofolate reductase, phenylalanine ammonia lyase, cystathionine beta synthase or hyaluronidase; preferably, wherein the small molecule is a small molecule drug, probe or dye, preferably, wherein the small molecule drug is doxorubicin or gemcitabine.

10. A conjugate, which contains the following formula: 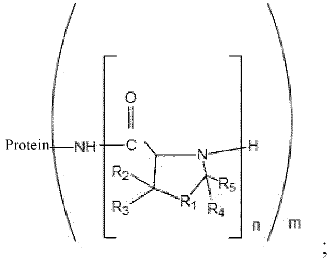 wherein R₁ is NH, CH₂, S, O or Se; R₂, R₃, R₄ and R₅ are each independently hydrogen, optionally substituted C₁-C₁₂ alkyl, optionally substituted C₂₋₆ alkenyl, optionally substituted C₂₋₆ alkynyl, optionally substituted C₁-C₁₂ heteroalkyl, C₂₋₆ heteroalkenyl, C₂₋₆ heteroalkynyl, halogen, hydroxyl, C₆-C₁₂ aryl, C₆-C₁₂ heteroaryl, -OC(=O)-optionally substituted C₁₋₆ alkyl, -OC(=O)-optionally substituted C₂₋₆ alkenyl, -OC(=O)-optionally substituted C₂₋₆ alkynyl, -OC(=O)-optionally substituted C₁₋₆ heteroalkyl, -OC(=O)-C₂₋₆ heteroalkenyl, -OC(=O)-C₂₋₆ heteroalkynyl, -S-C(=O)-optionally substituted C₁₋₆ alkyl, -S-C(=O)-optionally substituted C₂₋₆ alkenyl, -S-C(=O)-optionally substituted C₂₋₆ alkynyl, -S-C(=O)-optionally substituted C₁₋₆ heteroalkyl, -S-C(=O)-C₂₋₆ heteroalkenyl, -S-C(=O)-C₂₋₆ heteroalkynyl, -N₃, -C₁₋₆ alkyl-N₃, -C₁₋₆ heteroalkyl-N₃, -NHCOR', -NHCOOR' or -NR₁R₂, optionally substituted C₆-C₁₂ aryl, or optionally substituted C₆-C₁₂ heteroaryl, wherein R' is hydrogen, halogen, optionally substituted C₁₋₆ alkyl, optionally substituted C₂₋₆ alkenyl, optionally substituted C₂₋₆ alkynyl, optionally substituted C₁₋₆ heteroalkyl or optionally substituted C₆-C₁₂ aryl, R₁ and R₂ are each independently hydrogen, halogen, optionally substituted C₁₋₆ alkyl, optionally substituted C₂₋₆ alkenyl, optionally substituted C₂₋₆ alkynyl, optionally substituted C₁₋₆ alkoxy or optionally substituted C₆-C₁₂ aryl or optionally substituted C₆-C₁₂ heteroaryl; the protein is selected from enzymes, antibodies, cytokines and marker proteins, preferably asparaginase, uricase, phenylalanine ammonia lyase, cystathionine beta synthase, or hyaluronidase; wherein n is 1-200, and m is 1-X, wherein X is the number of reactive lysines on the protein, and/or the degree of dispersion of the polymer is 1-1.5.

11. The conjugate of claim 10, which is an asparaginase-polyproline conjugate or an uricase-polyproline conjugate, wherein m is 1-40.

12. A pharmaceutical composition comprising the conjugate of any one of claims 9-11 and a pharmaceutically acceptable carrier.

13. Use of the conjugate of any one of claims 9-11 or the pharmaceutical composition of claim 12 in the manufacture of a medicament.

14. The use of claim 13, wherein the medicament is for the treatment of cancer, such as lymphoma or leukemia, or for the treatment of hyperuricemia.
